# EUROPEAN PATENT APPLICATION

(11) **EP 4 112 097 A1**
(43) Date of publication of application: **04.01.2023**
(21) Application number: 21183125.0
(22) Date of filing: 01.07.2021
(51) Int. Cl.: A61M 5/20, A61M 5/24, A61M 5/315, A61M 5/32

(54) **AUTO INJECTOR AND CASSETTE SYSTEM WITH INDEX RING IN THE CASSETTE**

(71) Applicant: Phillips-Medisize A/S, 7600 Struer (DK)
(72) Inventor: LEMMING, Ole, 7600 Struer (DK); JENSEN, Peter, 2830 Virum (DK); JACOBSEN, Thorben Kvisgaard, 3230 Græsted (DK); JACOBSEN, Peter Blaabjerg, 7600 Struer (DK)
(74) Representative: Zacco Denmark A/S

(57) **Abstract**

The invention relates to a system comprising a cassette and an auto injector for delivery of medicament. The cassette for containing the medicament comprises an index ring.

## Description

The invention relates to a system comprising a cassette and an auto injector for delivery of medicament. The cassette for containing the medicament comprises an index ring.

### Background

Auto injectors for the delivery of medicament to a patient comes in many varieties depending on the type of medicament, which is to be delivered to the patient. When the auto injector is a re-usable injector part into which a disposable cassette is inserted, there are some main requirements, which need to be fulfilled for each of the two parts. For the auto injector, it needs to be ensured that all operational parts in the auto injector stay full functional in order to prolong the lifetime of the auto injector. For the cassette, there need to be mechanical stability, but at the same time, a minimum of material use in the disposable cassette is often also desired in order to keep production costs and the environmental impact low.

Patent application WO 2021/069106 A1 discloses an example of a system comprising a reusable auto injector and a disposable cassette, where the disposable cassette is absent of major electronic parts and spring systems. The auto injector comprises a number of injector pins, which moves into the cassette after assembly of the two parts thereby releasing a skin sensor and initiating a medicament delivery sequence. For releasing the skin sensor, flexible arms on the skin sensor deflects when the injector pins move into the cassette.

### Summary

Throughout this description, all references to the proximal direction or proximal surfaces refer to parts, surfaces and similar oriented in the direction of the injector site. Thus, by proximal end is meant the end of a part, which points towards the injection site. By proximal movement is meant a movement in a direction towards the injection site along/parallel with the longitudinal axis.

The distal end is the opposite end compared to the proximal end. Thus, all references to the distal direction or distal surfaces refer to parts, surfaces and similar oriented in the direction away from the direction of the injector site, i.e. in the direction of the user. Thus, distal movement is a movement in the opposite direction of the proximal movement. Proximal surfaces are surfaces pointing in the proximal direction, and distal surfaces are surfaces pointing the in distal direction.

Both the proximal and distal surfaces may be found at the distal end, the proximal end, or somewhere there in between, since the wording only refers to the direction in which the surfaces are oriented and not the position of the surfaces in the individual part.

Disclosed herein is in a **first aspect** is a cassette for use in an auto injector for administering a medicament, the cassette extending in a longitudinal direction from a proximal end to a distal end. The cassette comprises a syringe holder for receiving a syringe containing the medicament; a skin sensor longitudinally movable relative to the syringe holder between a plurality of longitudinal positions including at least a proximal longitudinal position, and a distal longitudinal position; and an index ring rotatable relative to the skin sensor between a plurality of rotational positions including at least a first rotational position in which the skin sensor is prevented from moving distally, and a second rotational position in which the skin sensor is moveable between the distal longitudinal position and the proximal longitudinal position; wherein the rotational position of the index ring is configured to be controlled by one or more release members in the auto injector when the cassette is secured in the auto injector.

By the cassette according to the first aspect is further obtained a cassette with an improved stability as compared to the cassette disclosed in WO 2021/069106 A1 due to the replacement of the fragile flexible arms on the skin sensor with the mechanical stable index ring. The index ring further allows for an improved control of the positions between which the skin sensor can move as a multiple of rotational positions of the index ring can be coupled to a multiple of longitudinal positions between which the skin sensor may be able to move. An improved control of skin sensor longitudinal position is further obtained. This provides an better safety of the user after insertion of a syringe with a needle in the cassette, since the user can be protected from coming into contact with the needle, which may be covered by the skin sensor.

Additionally, the manufacturing is easier and improved compared to previous known auto injectors.

Further, it is possible to obtain a more reliable and tamper proof detection by the cassette according to the first aspect, since if the locking ring is not in correct position, the cassette will be rejected by the auto injector. By the cassette according to the first aspect, full guidance in the device movements, instead of relying on plastic springs is obtained.

Disclosed herein in a **second aspect** is an auto injector for administering a medicament. The auto injector is adapted for receiving a cassette comprising a syringe holder for receiving a syringe containing medicament; a skin sensor longitudinally movable relative to the syringe holder between two or more longitudinal positions including at least a distal longitudinal position (LD) and a proximal longitudinal position (LP); and an index ring rotatable relative to the skin sensor between two or more rotational positions including a first rotational position (R1) and a second rotational position (R2) in which the skin sensor is moveable between the distal position and the proximal position.

The auto injector is extending from a proximal end to a distal end along a longitudinal axis, wherein the cassette is removable received in the auto injector along the longitudinal direction.

The auto injector comprises an injector housing and a multiple of internal injector parts positioned inside the injector housing, wherein the internal injector parts includes at least a plunger rod configured for proximal movement for delivery of medicament; a drive module adapted for moving the plunger rod proximately; and one or more release members configured for rotating the index ring between the two or more rotational positions when the one or more release members moves proximally; and a clutch configured for being movable between:
∘ a first clutch position where the plunger rod and the one or more release members are longitudinally coupled for proximal movement together; and
∘ a second clutch position where the plunger rod is decoupled from the one or more release members allowing the plunger rod to move longitudinally while the one or more release members are longitudinally stationary.

By the auto injector according to the second aspect is obtained an auto injector with an improved stability as compared to the auto injector disclosed in WO 2021/069106 A1 due to the replacement of the fragile pins with the more mechanical stable release member. The clutch introduced control of the plunger rod and the release member movement into the cassette, allows for a reduction of the material for the release member, since it need not follow the plunger rod all the way into the cassette during medicament delivery. Thereby more space for other components in the device is provided. Alternatively, the device is made more compact. This provides a reduction of the material cost for the cassette as well, since the cassette is not required to have compartments/space for pins extending along the entire length of the cassette, thereby providing a more compact and thinner cassette. Manufacturability of components and avoidance of over constrained end position is also obtained by the auto injector according to the second aspect. Also, less power and force loss from friction is obtained, as a reduced amount of friction when the power is needed for the injection is obtained.

Disclosed herein is in a **third aspect** is a cassette for use in an auto injector for administering a medicament, the cassette extending in a longitudinal direction from a proximal end to a distal end. The cassette comprises a syringe holder for receiving a syringe containing the medicament; a skin sensor comprising cap holding means at a proximal end of the skin sensor; a cassette housing comprising a cap holding part at a proximal end of the cassette housing; and a removable cassette cap comprising one or more locking cap parts extending distally from a proximal end of the cassette cap, wherein prior to use, a cassette cap locking protrusion on each of the one or more locking cap parts is secured inside the cassette housing. The skin sensor is longitudinally movable relative to the syringe holder between at least an initial locked position LP1 and an intermediate position LP2 being proximal of the initial locked position LP1, wherein upon proximal movement of the skin sensor from the initial locked position LP1 to the intermediate position LP2, the distal locking ends moves outside the cassette housing.

The intermediate position LP2 according to the **third aspect** may also be referred to as a ready-to-remove-cap position. By the cassette according to the **third aspect** is obtained a cassette with an improved control of the cassette cap removal as compared to the cassette disclosed in WO 2021/069106 A1. The cassette cap of the cassette in WO 2021/069106 A1 can be removed from the cassette prior to locking the cassette in an auto injector. The cassette according to the **third aspect,** may be constructed such that the cassette cap is prevented from being removed from the cassette until the cassette is secured firmly in an auto injector and an interaction with the auto injector has introduced a release of the skin sensor allowing the skin sensor to move to the intermediate position LP2 allowing for the cassette cap to be removed from the cassette. This improves the user safety. Additionally, drug safety is increased as the contact with a syringe inside the cassette is only available when intended to, i.e. when inserted correctly into an auto injector. If misuse of cassette has occurred this can be detected and the user will not be able to use the cassette. This reduces the risk of user being hurt by a needle in the syringe or the needle in the syringe being damaged by the user. Also, the drug barrier at the needle end is not compromised before use.

Disclosed herein is in a **fourth aspect** a system for administering a medicament, the system comprising a reusable auto injector according to the **second aspect** and a disposable cassette according to the **first aspect** or the **third aspect.**

Disclosed herein is in a **fifth aspect** a system for administration of medicament, the system comprising a cassette and an auto injector adapted for receiving the cassette. The cassette comprises a syringe comprising a syringe medicament compartment; an outlet at the proximal end of a syringe medicament compartment; and a stopper positioned inside the syringe compartment, wherein the stopper is movable from a distal end of the syringe compartment towards the proximal end of the syringe compartment for emptying the medicament in the syringe compartment through the outlet.

The cassette further comprises a syringe holder for receiving the syringe; and a skin sensor longitudinally movable relative to the syringe holder between two or more longitudinal positions selected from:
∘ a distal position LD;
∘ a final locked position LP3;
∘ an initial locked position LP1 between the final locked position LP3 and the distal position LD; and
∘ an intermediate position LP2 between the initial locked position LP1 and the final locked position LP3.

The cassette additionally comprises an index ring rotatable relative to the skin sensor between two or more rotational positions selected from:
∘ a first rotational position R1;
∘ a delivery rotational position Rd in which the skin sensor is moveable between the distal position LD and the final locked position LP3;
∘ a cap-release rotational position Rcr positioned between the first rotational position R1 and the delivery rotational position Rd.

The auto injector is extending from a proximal end to a distal end along a longitudinal axis, wherein the cassette is removable received in the auto injector along the longitudinal direction.

The auto injector comprises an injector housing and a multiple of internal injector parts positioned inside the injector housing, wherein the internal injector parts includes at least a plunger rod configured for proximal movement of the stopper inside the cassette; a drive module adapted for moving the plunger rod proximately; and one or more release members configured for rotating the index ring between the two or more rotational positions when the one or more release members moves proximally.

By the system according to the **fifth aspect** is obtained a system, which allows for an improved control of the cassette cap removal as compared to the cassette disclosed in WO 2021/069106 A1, where the cassette cap can be removed from the cassette prior to locking the cassette in an auto injector. This is not possible with the system according to the **fifth aspect,** where the cassette cap is locked inside the cassette housing until released by the auto injector. This increases the stability of the cassette and improves the user safety. Further, by the system according to the **fifth aspect,** is obtained a system with an improved stability as compared to the system disclosed in WO 2021/069106 A1 due to the replacement of the fragile flexible arms on the skin sensor in the cassette with the mechanical stable index ring. The index ring further allows for an improved control of the position between which the skin sensor can move as a multiple of rotational positions of the index ring can be coupled to a multiple of longitudinal positions between which the skin sensor may be able to move. By the system according to the fifth aspect is further obtained that the order of the step, which the user takes when using the system is controlled, which increases the safety for the patient using the system. Thus, the system controlled logic included in the reusable device ensures injection under improved safe conditions.

Disclosed herein in a **fifth aspect** is cassette for use in an auto injector for administering a medicament. The cassette comprising a syringe holder extending in a longitudinal direction from a proximal end to a distal end. The syringe holder comprises a syringe holder tubular part for receiving a syringe comprising the medicament; and a syringe holder lid.

The syringe holder tubular part comprises a plurality of syringe holder lid locking openings at a distal end of the syringe holder tubular part; wherein the holder lid comprises a substantially C-shaped lid part comprising an inner surface. The substantially C-shaped lid part is hingedly connected to the distal end of the syringe holder tubular part such that the substantially C-shaped lid part can be in at least two positions including:
∘ an open position allowing for a syringe to be positioned in the syringe holder through an opening in the distal end of the syringe holder tubular part, and
∘ a closed position preventing the syringe from exiting the syringe holder through the opening in the distal end of the syringe holder tubular part, and wherein the inner surface of the substantially C-shaped lid part is oriented towards the syringe when the syringe is positioned in the syringe holder.

The syringe holder lid further comprises a plurality of support lid parts extending from the inner surface of the substantially C-shaped lid part, and a plurality of lid locking tabs positioned in the plurality of syringe holder lid locking openings in the syringe holder tubular part when the substantially C-shaped lid part is in the closed position.

By the cassette with the syringe holder as described in the **fifth aspect** is obtained syringe holder having a more flexible lid. If the user presses on the syringe in a non-symmetrical manner, the syringe holder lid may only be affected in some parts and not over the entire lid surface. The breaking up of a ring-shaped lid thereby ensures that the forces are not transferred around the entire ring. This allows the lid to remain in a locked position inside the openings in the cylinder at more locations even if it is pressed out of position in one locations. Further, only the support lid parts protruding from the inner surface are allowed to come in contact with a syringe flange, which again ensures that forces are not transferred to the entire syringe holder lid.

The syringe normally only comes in contact with the syringe holder lid if it is pushed distally during use. There is thus normally an open space between the proximal surface of the syringe holder lid and the distal end of syringe. The syringe holder lid therefore has a safety function and not a syringe fixation or syringe holding function. The syringe is instead normally secured inside the syringe holder near the needle end by other means or along the tubular part of the syringe holder.

Normally, the auto injector according to any of the above aspects is a reusable injector, and the cassette according to any of the above aspects are a disposable cassette, which is disposed of after medicament delivery. The cassette may be compatible with a range of standard prefilled syringes and may thus be used to deliver wide range of drug products with very different injection volumes and viscosities.

Typically, the cassette is made of plastic only and therefore lacks metal springs. This reduces the size and the weight of the cassette compared to known disposable injectors also including injector parts for controlling and moving the medicament out of the syringe. During transport and storage, the cassette is not under any load, allowing for a minimal approach to packaging, and reduction of the requirements for this. Thus by ensuring that the cassette is absent of springs, improved mechanical stability during transportation is ensured.

A syringe may be mounted in the cassette by axial insertion of the syringe at the distal end of the cassette. The syringe suitable for being inserted in the cassette may comprise a syringe compartment containing the medication, an outlet at a proximal end of a syringe compartment, and a stopper positioned inside the syringe compartment. The stopper is normally movable from a distal end of the syringe compartment towards the proximal end of the syringe compartment for emptying the medicament in the syringe compartment through the outlet. When a syringe is positioned in the syringe holder, the skin sensor may cover the syringe outlet when the skin sensor is in the proximal longitudinal position, whereas the syringe outlet is exposed when the skin sensor is in the distal longitudinal position.

Normally, the syringe is a 1,0 mL or a 2,25 mL syringe. The syringe will normally have a shoulder part tapering at a proximal end of the syringe compartment towards the syringe outlet. Also, normally a flange is found at the distal end of the syringe compartment.

When reference is made to a 'proximal longitudinal position' in connect with the skin sensor in the cassette according to any of the above aspect, a multiple of proximal positions may be referred to. Each of these positions normally may be representing positions where the skin sensor covers the outlet. The proximal positions may include an initial position LP1, an intermediate position LP2, and a final position LP3. The initial position LP1 is a position distally of the final position LP3 and proximally of the distal position LD, thus being positioned between the two. The intermediate position LP2 is a position between the initial position LP1 and the final position LP3.

The proximal positions may be locked positions, from where proximal and/or distal movement of the skin sensor is prevented. Thus, the longitudinal positions may also be referred to as locked positions. Whether a longitudinal position is locked or not is determined by the position of the index ring. When the index ring is the first rotational position, the skin sensor is normally in a locked longitudinal proximal position irrespectively of which of the longitudinal proximal positions the skin sensor is in.

When reference is made to a 'first rotational position' in connect with the index ring in the cassette according to any of the above aspects, it will normally refer to a position from where rotation in only one direction is possible. However, the second rotational position need not be a position from where the index ring cannot rotate further away from the first rotational position. The second rotational position may also be an intermediate position in which the skin sensor is unlocked, but in which the skin sensor is still prevented from moving to the most proximal position and/or the most distal position possible. The second rotational position may for example be a cap-release rotational position or a delivery rotational position.

When the index ring is rotated from the second rotational position to the first rotational position, the skin sensor may be locked in the longitudinal direction by interaction with the index ring. The skin sensor may be locked in the longitudinal position, which the skin sensor is in at the point in time when the index ring is rotated from the second rotational position to the first rotational position. The longitudinal position of the skin sensor may be determined by user interaction with the cassette, e.g. pushing the skin sensor distally in the longitudinal direction. The locked position may thus be an initial locked position, a final locked position after medicament injection, or an intermediate locked position, in which the skin sensor may be locked if the medicament delivery procedure is abandoned before the medicament has been fully injected.

Thus, in one or more examples, when the skin sensor is in the proximal longitudinal position, and the index ring is in the first rotational position, the skin sensor is prevented from moving distally from the proximal longitudinal position by the index ring.

Prior to use, the index ring may be in the first rotational position and the skin sensor may be locked relative to the syringe holder in an initial locked position. As above, the initial locked position is an example of a proximal position. By 'prior to use' is meant the initial delivery configuration, which the cassette has prior to being assembled with an auto injector. The locking of the skin sensor and the index ring ensures that the skin sensor is locked in position longitudinally and that the index ring is locked in position rotationally.

In one or more examples, when the index ring is in the second rotational position, the skin sensor is unlocked and able to move relative to the syringe holder in a proximal direction and/or a distal direction. Thus, during the rotation of the index ring from the first position to the second position, the skin sensor is unlocked from the initial locked position allowing it to move longitudinally in the distal and/or proximal direction. The release may occur at any point during the rotation of the index ring. Normally, the index ring is rotated an amount before the release occurs ensuring that the release cannot occur by e.g. vigorous shaking of the cassette. The distance in the longitudinal direction, which the skin sensor is allowed to move when released, may vary depending on how much the index ring is rotated compared to the initial first rotational position. Normally, when the skin sensor is unlocked from the initial locked position allowing it to move longitudinally in both the distal and the proximal direction.

In one or more examples, the index ring is symmetric around the longitudinal axis such that a 180 degree rotation of the index ring provides the same design. This gives the user the option to insert the cassette in the auto injector in two different manners, which gives an increased insertion flexibility of the cassette internal parts also, but further helps to ensure that an forces from the outside will not move the index ring operational state.

In one or more examples, prior to use, the skin sensor is locked in a initial locked position, and after use, the skin sensor is in a final locked position, wherein the initial locked position is positioned between a final locked position and the distal longitudinal position.

The initial locked position and the final locked position are both examples of a proximal position. By 'prior to use' is meant the initial delivery configuration, which the cassette has prior to being assembled with an auto injector. By 'after use' is meant configuration, which the cassette is in after full delivery of the medicament. In the 'after use' configuration, the needle inserted in the user during medicament delivery has been removed from the injection site.

In one or more examples, the cassette further comprises a cassette housing and a removable cassette cap positioned at a proximal end of the cassette housing. Normally, the cassette housing at least partly encloses the syringe holder, the syringe when positioned in the syringe holder, the skin sensor, and the index ring at least prior to use. Often the cassette housing will enclose at least 90% of the syringe holder, the skin sensor, and the index ring if not fully enclosing the parts.

In one or more examples, the cassette cap comprises one or more cap locking parts extending distally from a proximal end of the cassette cap, wherein prior to use, a cassette cap locking protrusion on each of the one or more cap locking parts is secured inside the cassette housing. The skin sensor may also comprise cap holding means, the cap holding means including a recess and ramp at the proximal end of the skin sensor, the recess being positioned distally of the ramp. The cassette housing may be comprising a cap holding part extending radially inwardly at the proximal end of the cassette housing. Prior to removal of the cassette cap, an inwardly extending part of the cassette cap locking protrusion on each of the one or more cap locking parts may be secured between the recess of the skin sensor and the cap holding part, and further be abutting the proximal protruding part preventing proximal movement of the cassette cap. This firmly secures the cassette cap inside the cassette between the skin sensor and the cassette housing.

In one or more examples, upon proximal movement of the skin sensor from the initial locked position to the intermediate position, the distal locking ends are moved to a position outside the cassette housing, and wherein when the skin sensor is in the intermediate position, removal of the cassette cap is possible. The removal of the skin sensor may be performed at this point without exerting a large force, but instead by simply pulling the cassette cap in the proximal direction. If a syringe with a needle shield, e.g. a rigid needle shield (RNS), is included in the cassette, the removal of the cassette cap will normally also introduce a removal of the needle shield, as the cassette cap is normally fitted around the needle shield such that the two parts move together. The cassette cap may comprise a number of subparts depending on and fitted to the type of needle shield to be removed.

In one or more examples, the one or more cap locking parts and/or the cassette cap locking protrusion on each of the one or more cap locking part are flexible, wherein upon proximal movement of the skin sensor from the initial locked position LP1 to the intermediate position LP2, the one or more cap locking parts and/or the cassette cap locking protrusion on the locking part flex radially inwards in the recess of the skin sensor thereby enabling the cassette cap locking protrusions to move proximally pass the cap holding part, wherein when the skin sensor is in the intermediate position LP2, the one or more locking parts and/or the cassette cap locking protrusion on the locking part are configured to flex radially outwardly and slide up the ramp upon removable of the cassette cap from the cassette.

After end of medicament delivery, the cassette cap may be attached to the cassette again. The cassette cap will normally not be locked between the cassette housing and the skin sensor as in the initial delivery position, but it may be positioned in the recess of the skin sensor.

In one or more examples, the cassette housing at least partly encloses the syringe holder and the skin sensor. Often the cassette housing will enclose at least 90% of the syringe holder and the skin sensor if not fully enclosing the syringe holder and the skin sensor.

In one or more examples, the cassette further comprises an index ring rotatable relative to the skin sensor between at least a first rotational position R1, and a cap-release rotational position Rcr, wherein prior to use, the index ring is in the first rotational position R1, and the skin sensor is in the initial locked position LP1, wherein when the index ring is in the cap-release rotational position Rcr, the skin sensor is movable to the intermediate position LP2, wherein the rotational position of the index ring is controlled by locking members in the auto injector when the cassette is secured in the auto injector.

In one or more examples, the index ring is further rotatable to a delivery rotational position Rd, wherein the cap-release rotational position Rcr is positioned between the first rotational position R1 and the delivery rotational position Rd, wherein when the index ring is in the delivery rotational position Rd, the skin sensor is movable between a final locked position LP3 and a distal longitudinal position LD; wherein the initial locked position LP1 and the intermediate position LP2 are positioned between the final locked position LP3 and the distal longitudinal position LD.

In one or more examples, the syringe outlet comprises a needle covered by a removable needle shield, and wherein the cassette cap comprises a second distally extending part extending around and grapping the needle shield to remove the needle shield when the cassette cap is removed from the cassette.

In one or more examples, the cassette further comprises a syringe having a syringe compartment containing the medicament; an outlet at a proximal end of the syringe compartment; and a stopper positioned inside the syringe compartment, wherein the stopper is movable from a distal end of the syringe compartment towards the proximal end of the syringe compartment for emptying the medicament in the syringe compartment through the outlet. The syringe outlet may be a hollow needle.

In one or more examples, the syringe comprises a removable needle shield covering the syringe outlet prior to use, and wherein the cassette cap comprises a second distally extending part extending around and grapping the needle shield, thereby removing the needle shield when the cassette cap is removed from the cassette housing.

In one or more examples, when the skin sensor is in the distal longitudinal position LD, the syringe outlet is exposed, and wherein the skin sensor covers the syringe outlet when the skin sensor is in either of the initial locked position LP1; the intermediate position LP2; and the final locked position LP3. The initial locked position LP1, the intermediate position LP2, and the final locked position LP3 are all examples of proximal longitudinal positions.

Thus, in one or more examples, when the skin sensor is in the final locked position and the index ring is in the first rotational position, the skin sensor covers the syringe outlet. Also, in one or more examples, when the skin sensor is in the distal longitudinal position and the index ring is in the delivery rotational position, the syringe outlet is exposed.

In one or more examples, medicament can be delivered by an auto injector induced proximal movement of the stopper inside the syringe compartment when the skin sensor is in the distal longitudinal position LD and the index ring is in the delivery rotational position Rd.

The skin sensor may be aligned with a cassette housing at the proximal ends of the two when in the skin sensor is in the distal longitudinal position LD. The distal longitudinal position LD may also be seen as a delivery position. Normally, the skin sensor is pushed into the distal longitudinal position LD when the user presses the proximal end of the cassette against the delivery site. The skin sensor is therefore normally not locked in the distal longitudinal position LD. The position is instead maintained by the user pressing the proximal end of the cassette against the delivery site. If the user removes the cassette from the injection site prior to completion of medicament delivery, the skin sensor will normally be moved proximally from the distal longitudinal position LD due to an interaction with the auto injector. This will normally move the skin sensor into a position where the syringe outlet is covered by the skin sensor.

In one or more examples, the index ring is configured for moving from the cap-release rotational position Rcr to the delivery rotational position Rd preparing the system for medicament delivery when the skin sensor is in the distal longitudinal position LD.

In one or more examples, the index ring comprises a tubular index ring part extending around a tubular skin sensor part. Alternatively or in combination, the index ring may comprise a tubular index ring part extending around the syringe holder.

In one or more examples, the index ring comprises a track extending in a sloping direction, and wherein each of the one or more release members is configured for traveling inside the track, and wherein the index ring is rotated when the release member travels proximally inside the track. Each of the one or more release members may comprise a track-guide protrusion, wherein the track-guide protrusion is configured for traveling inside the track.

In one or more examples, the index ring index ring comprises a track extending from a first corner to a second corner, wherein the index ring is configured for rotating from the first rotational position to the second rotational position when the cassette is inserted in the auto injector and a release member comprised in the auto injector travels proximally inside the track.

In one or more examples, the index ring comprises a first radially extending part having a first surface area extending between a first proximal surface and a first distal surface, wherein the first surface area comprises a track extending from a first corner of the surface area to a second corner of the surface area, wherein the index ring is configured for rotating from the first rotational position to the second rotational position when the cassette is inserted in the auto injector and a release member comprised in the auto injector travels proximally inside the track. Thus, the index ring may comprise a first radially extending part having a first surface area extending between a first proximal surface and a first distal surface, wherein the track is positioned on the surface area.

The surface area on the radial part need not cover the entire surface of the radial part, but can instead be a smaller area, which is defined by the by the two corners between which the track extends.

The first radially extending part may be part of the tubular index ring part.

In one or more examples, the track is positioned on the first longitudinally extending part.

In one or more examples, the index ring is configured for rotating to the first rotational position when the release member comprised in the auto injector travels distally inside the track. Each of the one or more release members may comprise a track-guide protrusion, wherein the track-guide protrusion is configured for traveling inside the track. By retrieving the release member / track-guide protrusion into the auto injector again by distal movement of the track-guide protrusion, the index ring is rotated back to its initial position. The rotational position of the index ring is therefore controlled by the release member movement.

In one or more examples, the track extends from the first corner in an angled direction towards the second corner, wherein the angled directions is between 20 and 70 degrees, such as between 30 and 60 degrees, such as between 35 and 55 degrees, such as between 40 and 50 degrees, such as approximately 45 degrees relative to the longitudinal direction of the cassette.

In one or more examples, the track comprises a first track section, a second track section and a third track section, wherein:
∘ the first track section extends from the first corner in a first angled direction towards a first middle point;
∘ the second track section extends from the first middle point to a second middle point along the longitudinal direction of the cassette in a second angle direction;
∘ the third track section extends from the second middle point in a third angled direction towards the second corner,
wherein the first and/or third angled directions are between 20 and 70 degrees, such as between 30 and 60 degrees, such as between 35 and 55 degrees, such as between 40 and 50 degrees, such as approximately 45 degrees relative to the longitudinal direction of the cassette, and
wherein the second angled directions is between -20 and 20 degrees, such as between -10 and 10 degrees, such as approximately 0 degrees relative to the longitudinal direction of the cassette.

Alternative track slopes may also be imagined, where the sloping angle changes in a continuous manner in any of the angled directions such that the track section(s) obtains a concave or convex track slopes.

In one or more examples, the skin sensor comprises a protruding tab on an outer surface of the skin sensor, and the index ring comprises a first longitudinally extending part with a first recess in which the protruding tab is confined prior to use.

In one or more examples, the index ring comprises a locking tab, and the skin sensor comprises a first locking recess in which locking tab is confined prior to use.

In one or more examples, the first longitudinally extending part extends proximally from the first proximal surface on the first radially extending part.

In one or more examples, the protruding tab abuts the first proximal surface when the skin sensor is in the distal longitudinal position LD thereby preventing distal movement of the skin sensor relative to the syringe holder.

In one or more examples, the skin sensor comprises a first distal surface, wherein the first distal surface abuts the first proximal surface when the skin sensor is in the distal longitudinal position LD thereby preventing distal movement of the skin sensor relative to the syringe holder.

In one or more examples, the index ring further comprises a second radially extending part having a second proximal surface and a second distal surface.

In one or more examples, the protruding tab is abutting the second proximal surface when the skin sensor is in the final locked position LP3 and the index ring is in the first rotational position R1 thereby preventing distal movement of the skin sensor relative to the syringe holder.

In one or more examples, the distal surface is abutting the second proximal surface when the skin sensor is in the final locked position LP3 and the index ring is in the first rotational position R1 thereby preventing distal movement of the skin sensor relative to the syringe holder.

In one or more examples, the second radially extending part is connected to a proximal end of the first longitudinally extending part.

In one or more examples, the index ring comprises a second longitudinally extending part extending from the first proximal surface of the first radially extending part and connected to the second radially extending part at a proximal end of the second longitudinally extending part.

In one or more examples, the index ring is configured for being rotated to a cap-release rotational position Rcr positioned between the first rotational position R1 and a delivery rotational position Rd, wherein in the cap-release rotational position Rcr the skin sensor is movable between the distal longitudinal position LD and an intermediate position LP2, wherein the intermediate position LP2 is positioned between the final locked position LP3 and the initial locked position LP1. The intermediate position is an example of a proximal position.

In one or more examples, the index ring is in the cap-release rotational position Rcr when the release member comprised in the auto injector is in the second track section.

In one or more examples, when the index ring is in the cap-release rotational position Rcr, the skin sensor is prevented from moving from the intermediate position LP2 to the final locked position LP3. Preventing proximal movement from the intermediate position PL2 may be obtained in a number of ways.

In one or more examples, when the index ring is in the cap-release rotational position Rcr and the skin sensor is in the intermediate position LP2, the protruding tab abuts the second distal surface on the index ring. The skin sensor is hereby preventing from moving proximally from the intermediate position LP2.

In one or more examples, when the index ring is in the cap-release rotational position Rcr and the skin sensor is in the intermediate position, the locking tab on the index ring abuts a second locking tab support surface on the skin sensor. The skin sensor is hereby preventing from moving proximally from the intermediate position LP2.

In one or more examples, the skin sensor is configured for being moved to the distal longitudinal position LD by a user pushing the skin sensor against an injection site when the index ring is in the cap-release rotational position Rcr, between the cap-release rotational position Rcr and the delivery rotational position Rd, or in the delivery rotational position Rd.

In one or more examples, the first longitudinally extending part comprising a second recess in which the protruding tab is confined when the skin sensor is in the intermediate position LP2 and the index ring is in the first rotational position R1.

In one or more examples, the protruding tab on the first longitudinally extending part is confined in a second locking recess on the skin sensor when the skin sensor is in the intermediate position LP2 and the index ring is in the first rotational position R1.

In one or more examples, the index ring is longitudinally locked relative to the syringe holder. Likewise, the skin sensor and the syringe holder are normally rotationally locked.

The cassette may comprise a label such as e.g. an RFID tag readable by the auto injector. The information contained on the RFID tag may be relating to the type of medicament inside the cassette or the medicament dose. The auto injector may be configured for reading the information on the RFID tag and control the movement of a plunger rod for pushing the stopper proximally for medicament delivery accordingly.

The syringe holder as described above in the fifth aspect is suitable for use with any of the described examples of the cassette. In one or more examples of the syringe holder, the substantially C-shaped lid part comprises a throughgoing channel in the substantially C-shaped lid part dividing the substantially C-shaped lid part into two lid sections, including a first lid section and a second lid section. The C-shaped lid part may also be referred to as an open circle or a circle with a cut-through opening in the circumference. The substantially C-shaped lid part may thus have the shape of a circle with an opening spanning an area of a full circular circumference of the circle being at least 10%, such as at least 20%, such as at least 30%.

The two lid sections may be connected by a bridge portion of substantially C-shaped lid part also forming the hinged connecting to the distal end of the syringe holder tubular part. As an alternative to a C-shaped lid, a two part lid formed as two half circles may also be imagined. The two half circles may be connected by a bridge portion or alternatively, each of the separate half circles may be hingedly connected to the syringe holder tubular part.

In one or more examples of the syringe holder, the first lid section and the second lid section are at least partly displaceable in relation to each other along the longitudinal axis. By partly displaceable is meant that one section may be twisted or pushed distally or proximally without moving the other section to the same extend. The flexibility in the substantially C-shaped lid part further ensures that if the user pushes on the syringe unevenly from a proximal direction, only part of the substantially C-shaped lid part is affected since the forces are not easily transferred around in the substantially C-shaped lid part.

In one or more examples of the syringe holder, the plurality of support lid parts covers an area of the inner surface on the substantially C-shaped lid part being less than 50%, such as less than 40%, such as less than 30%, such as less than 20%, such as less than 10%. Having a broken surface structure on the surface, which the syringe may come in contact with ensures that if the user pushes on the syringe unevenly from a proximal direction, not all of the support lid parts will come in contact with the syringe. This ensures the pushing forces are not transferred around in the entire substantially C-shaped lid part. If a user should succeed in pushing so hard on the syringe that one of the locking tabs are pushed out of the syringe holder lid locking opening, there is an increased change that the other locking tabs will remain in position thereby providing an increased security preventing the syringe from being pushed out of the distal end of the syringe holder.

In one or more examples of the syringe holder, the plurality of support lid parts includes at least four support lid parts. Each of the plurality of lid locking tabs may fit into a separate one of the syringe holder lid locking openings. The distal surface of the lid locking tabs may rest against a proximal inner surface in each of the syringe holder lid locking openings when the syringe holder lid is in a closed position.

In one or more examples of the syringe holder, when a syringe is correctly positioned inside the syringe holder tubular part and the cassette is ready for use, the syringe is not in direct contact with the syringe holder lid. The syringe normally only comes in contact with the syringe holder lid if it is pushed distally during use. There is thus normally an open space between the proximal surface of the syringe holder lid and the distal end of syringe. The syringe holder lid therefore has a safety function and not a syringe fixation or syringe holding function.

In one or more examples a syringe is enclosed in the syringe holder, wherein the syringe is comprising:
∘ a syringe compartment containing the medicament;
∘ an outlet at a proximal end of the syringe compartment;
∘ a stopper positioned inside the syringe compartment, wherein the stopper is movable from a distal end of the syringe compartment towards the proximal end of the syringe compartment for emptying the medicament in the syringe compartment through the outlet; and
∘ a syringe flange at a distal end of the syringe compartment.

In one or more examples of the cassette with the syringe holder as described above, the cassette further comprising a skin sensor movable relative to the syringe holder between a plurality of longitudinal positions including at least a proximal longitudinal position; and a distal longitudinal position.

In one or more examples of the cassette with the syringe holder as described above, the cassette is comprising an index ring rotatable relative to the skin sensor between a plurality of rotational positions including at least a first rotational position in which the skin sensor is prevented from moving distally, and a second rotational position in which the skin sensor is moveable between the distal longitudinal position and the proximal longitudinal position, wherein the rotational position of the index ring is configured to be controlled by one or more release members in the auto injector when the cassette is secured in the auto injector.

In one or more examples of the cassette with the syringe holder as described above, the cassette is further comprising a cassette housing comprising a cap holding part at a proximal end of the cassette housing; and a removable cassette cap secured inside the cassette housing.

In one or more examples of the cassette with the syringe holder as described above, the cassette housing comprises a cap holding part at a proximal end of the cassette housing; and wherein the removable cassette cap comprises one or more locking cap parts extending distally from a proximal end of the cassette cap, wherein prior to use, a cassette cap locking protrusion on each of the one or more locking cap parts is secured inside the cassette housing, wherein the skin sensor is longitudinally movable relative to the syringe holder between at least an initial locked position and an intermediate position being proximal of the initial locked position, and wherein upon proximal movement of the skin sensor from the initial locked position to the intermediate position, the distal locking ends moves outside the cassette housing.

In one or more examples of the auto injector, the injector housing comprises a cassette covering section extending to cover at least a part of the cassette when received inside the injector; and a distal housing section extending to cover the internal injector parts inside the injector housing before the cassette is secured inside the injector.

When the cassette is received inside the injector, the cassette covering section extends to cover 20-90% of the cassette, such as 30-75% of the cassette, such as 40-60% of the cassette. The auto injector comprises a cassette abutting surface adapted for abutting a distal end of the cassette when secured inside the auto injector.

In one or more examples of the auto injector comprises one or more cassette interacting parts configured for engaging in a snap-fit connection with corresponding one or more cassette securing tabs on the cassette when the cassette is inserted into the injector, and wherein proximal movement of the release member prevents a disengagement of the snap-fit connection thereby securing the cassette inside the auto injector.

In one or more examples of the auto injector, the skin sensor spring system is adapted for pushing the cassette skin sensor in a proximal direction to the proximal longitudinal position LP2 when the index ring is in the cap-release rotational position Rcr.

In one or more examples of the auto injector, the skin sensor spring system is adapted for pushing the cassette skin sensor in a proximal direction to the proximal longitudinal position when the index ring is in the second rotational position R2.If the skin sensor is pressed against an injection site, the skin sensor will be forced into / towards the distal longitudinal position LD. However, if the skin sensor is removed from the skin, the skin sensor spring system in the auto injector will push the skin sensor into a proximal longitudinal position when the index ring is in the second rotational position R2. This is true independently of whether the index ring is in the cap removal rotational position Rcr or the delivery rotational position Rd.

In one or more examples of the auto injector, after medicament delivery and removal of the system from the delivery site, the skin sensor spring system is configured for keeping the skin sensor in the final longitudinal position LP3 during distal movement of the release member whereby the index ring is rotated from the delivery rotational position Rd to the first rotational position R1.

In one or more examples of the auto injector, the index ring is in the first rotational position R1 prior to use and wherein when the index ring is moved from the first rotational position R1 to the cap-release rotational position Rcr by proximal movement of the one or more release members, the skin sensor spring system is adapted for pushing the skin sensor to the intermediate position LP2.

In one or more examples of the auto injector, the internal injector parts further includes a clutch configured for being movable between a first clutch position where the plunger rod and the one or more release members are longitudinally coupled for proximal movement together; and a second clutch position where the plunger rod is decoupled from the one or more release members allowing the plunger rod to move longitudinally while the one or more release members are longitudinally stationary.

In one or more examples of the auto injector, the one or more release members are coupled to the injector housing in the second clutch position.

In one or more examples of the auto injector, the clutch is in the first clutch position prior to use and wherein the clutch is configured for moving from the first clutch position to the second clutch position when the index ring is rotated to the second rotational position R2 by proximal movement of the one or more release members.

In one or more examples of the auto injector, the clutch is configured for remaining in the second clutch position when the index ring is in the second rotational position R2 and the plunger rod moves further proximally for delivery of the medicament.

In one or more examples of the auto injector, the clutch is configured for moving from the second clutch position to the first clutch position when the plunger rod moves distally after medicament delivery whereby the index ring is rotated from the second rotational position R2 towards the first rotation position R1 by distal movement of the one or more release members when the clutch is in the first clutch position.

In one or more examples of the auto injector, the clutch is a tubular clutch having one or more clutch tabs with a first rotation surface and a second rotation surface, and the internal injector parts comprises a proximal chassis part having a clutch coupling part with one or more first coupling surfaces and one or more second coupling surfaces, wherein:
- when the second rotation surface on the clutch is abutting the second coupling surfaces on the clutch coupling part, the clutch is in the second clutch position; and
- when the second rotation surface on the clutch is not in contact with the second coupling surfaces on the clutch coupling part, the clutch is in the first clutch position.

In one or more examples of the auto injector, the plunger rod is configured for moving in the longitudinal direction relative to the clutch, wherein the clutch is configured for:
- moving proximally with the one or more release members before the second rotation surface comes into contact with the second coupling surfaces;
- axial rotation when the second rotation surface comes into contact with the second coupling surfaces, thereby moving from the first clutch position to the second clutch position.

In one or more examples of the auto injector, the clutch coupling part comprises a clutch stopping surface abutting the clutch when the clutch is in the second clutch position thereby preventing the clutch and the one or more release members from moving longitudinally with the plunger rod.

In one or more examples of the auto injector, the plunger rod comprises a stopping clutch tab, wherein a distal surface of the stopping clutch tab is abutting a proximal surface of the one or more release members when the clutch is in the first clutch position.

In one or more examples of the auto injector, the stopping clutch tab is configured for moving the clutch from the second clutch position to the first clutch position upon distal movement of the plunger rod bringing the distal surface of the stopping clutch tab in contact with the proximal surface of the one or more release members.

In one or more examples, the cassette further comprises a cassette housing comprising a cap holding part at a proximal end of the cassette housing, and a removable cassette cap comprising one or more locking cap parts extending distally from a proximal end of the cassette cap, wherein prior to use, a cassette cap locking protrusion on each of the one or more locking cap parts is secured inside the cassette housing, wherein upon proximal movement of the skin sensor from the initial locked position LP1 to the intermediate position LP2, the distal locking ends moves outside the cassette housing.

From a sustainability perspective, embedding any type of metal parts and/or electronic components into a disposable device, is a non-ideal solution. Likewise, from a usability perspective, reusing parts of the cassette is also a non-ideal solution, as it adds user steps, and no guarantee for actual use. The auto injector and cassette combination described herein therefore provides a very good overall solution, optimizing both sustainability and usability.

In one or more examples, the auto injector further comprises a processor controlling the drive module.

In one or more examples, the drive module further comprises gear wheels and a motor configured for rotating a threaded rod inside the plunger rod thereby driving the plunger rod longitudinally.

In one or more examples, the processor is configured for controlling an applied force applied on the stopper in the syringe compartment.

All aspects of the plunger movement are controllable from parameter settings in the firmware of the device and can be configured exactly to the specific requirements from the therapy. An advantage of the motor drive is that high forces can be applied in a fully controlled process, so it is possible to optimize delivery of even highly viscous drugs, and still minimize the needle size.

In one or more examples, the drive module further comprises a battery, such as a rechargeable battery, wherein the rechargeable battery is adapted to be chargeable, such as chargeable from a USB charger. The battery may be a Li-ion cell battery.

The auto injector may further comprise Bluetooth low energy (BLE) connectivity options, which may convey selected information on e.g. the plunger rod position to an app, and a secure back-end. A user interfaces with LED's and/or audible support and/or displays may be included in the auto injector.

It is noted that all cassette and auto injector details described above applies to all described aspects of the cassette, auto injector and system comprising both parts.

### Brief description of the drawings

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example needs not have all the aspects or advantages shown. An aspect or an advantage described in conjunction with a particular example is not necessarily limited to that example and can be practiced in any other examples even if not so illustrated, or if not so explicitly described.
Figures 1a-b show exploded views of a first example of a cassette extending along a longitudinal direction L, and figure 1c show the cassette housing in a side view.
Figures 2a-c show an auto injector configured for receiving a cassette as shown in figures 1a-c, where figure 2a shows the assemble injector, figure 2b shows a view of the surface in the auto injector in contact with the cassette, and figure 2c shows an exploded view of the auto injector.
Figure 3a show the plurality of longitudinal positions which the skin sensor may move into relative to the syringe holder, and figure 3b shows the rotational positions which the index ring may be rotated between.
Figures 4a-i show the syringe holder, the skin sensor, and the index ring of the cassette from figures 1a-b, and the injector release member shown in figures 2c and 11a-d in different skin sensor longitudinal positions and index ring rotational positions as the injector release member moves longitudinally in the index ring track.
Figures 5a-c show the assembly of the syringe holder in the cassette as shown in the previous figures.
Figures 6a-i show a second example of the syringe holder, the skin sensor, and the index ring, and the injector release member in different skin sensor longitudinal positions and index ring rotational positions as the injector release member moves longitudinally in the index ring track.
Figures 7a-b show a third example of the syringe holder, the skin sensor, and the index ring and figure 7c shows the cassette housing in a third example.
Figures 8a-g show the syringe holder, the skin sensor, and the index ring of figures 7a-b, and the injector release member in different skin sensor longitudinal positions and index ring rotational positions as the injector release member moves longitudinally in the index ring track, and figures 8h-i show a cut-through of the cassette housing and the skin sensor showing the skin sensor in non-locked position (figure 8h) and the final position LP3 (figure 8i).
Figures 9a-d show the removal of the cassette cap from the cassette.
Figures 10a-d show the clutch coupling parts in different configurations during medicament delivery.
Figures 11a-d show a simplified version of figures 10a-d.
Figures 12a-b show the clutch ring, and figures 12c-d show the injector chassis with the clutch interacting part.
Figures 13a-e show the locking of the cassette in the auto injector.
Figures 14a-e show an example of a syringe holder suitable for use with any of the above described examples of the cassette.
Figures 15a-d show the syringe holder of figures 14a-e with a syringe positioned inside the syringe holder.

### Description of examples

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

In the drawings, thicknesses of a plurality of layers and areas are illustrated in an enlarged manner for clarity and ease of description thereof. When a layer, area, element, or plate is referred to as being "on" another layer, area, element, or plate, it may be directly on the other layer, area, element, or plate, or intervening layers, areas, elements, or plates may be present therebetween. Conversely, when a layer, area, element, or plate is referred to as being "directly on" another layer, area, element, or plate, there are no intervening layers, areas, elements, or plates therebetween. Further when a layer, area, element, or plate is referred to as being "below" another layer, area, element, or plate, it may be directly below the other layer, area, element, or plate, or intervening layers, areas, elements, or plates may be present therebetween. Conversely, when a layer, area, element, or plate is referred to as being "directly below" another layer, area, element, or plate, there are no intervening layers, areas, elements, or plates therebetween.

The spatially relative terms "lower" or "bottom" and "upper" or "top", "below", "beneath", "less", "above", and the like, may be used herein for ease of description to describe the relationship between one element or component and another element or component as illustrated in the drawings. It will be understood that the spatially relative terms are intended to encompass different orientations of the device in use or operation, in addition to the orientation depicted in the drawings. For example, in the case where a device illustrated in the drawings is turned over, elements described as being on the "lower" side of other elements, or "below" or "beneath" another element would then be oriented on "upper" sides of the other elements, or "above" another element. Accordingly, the illustrative term "below" or "beneath" may include both the "lower" and "upper" orientation positions, depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below, and thus the spatially relative terms may be interpreted differently depending on the orientations described.

Throughout the specification, when an element is referred to as being "connected" to another element, the element is "directly connected" to the other element, or "electrically connected" to the other element with one or more intervening elements interposed therebetween.

The terminology used herein is for the purpose of describing particular examples only and is not intended to be limiting. As used herein, the singular forms "a," "an," and "the" are intended to include the plural forms, including "at least one," unless the content clearly indicates otherwise. "At least one" is not to be construed as limiting "a" or "an." It will be further understood that the terms "comprises," "comprising," "includes" and/or "including," when used in this specification, specify the presence of stated features, integers, steps, operations, elements, and/or components, but do not preclude the presence or addition of one or more other features, integers, steps, operations, elements, components, and/or groups thereof.

It will be understood that, although the terms "first," "second," "third," and the like may be used herein to describe various elements, these elements should not be limited by these terms. These terms are only used to distinguish one element from another element. Thus, "a first element" discussed below could be termed "a second element" or "a third element," and "a second element" and "a third element" may be termed likewise without departing from the teachings herein.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

Exemplary examples are described herein with reference to cross track section illustrations that are schematic illustrations of idealized examples, wherein like reference numerals refer to like elements throughout the specification. As such, variations from the shapes of the illustrations as a result, for example, of manufacturing techniques and/or tolerances, are to be expected. Thus, examples described herein should not be construed as limited to the particular shapes of regions as illustrated herein but are to include deviations in shapes that result, for example, from manufacturing. For example, a region illustrated or described as flat may have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims. Some of the parts which are not associated with the description may not be provided in order to specifically describe exemplary examples of the present disclosure.

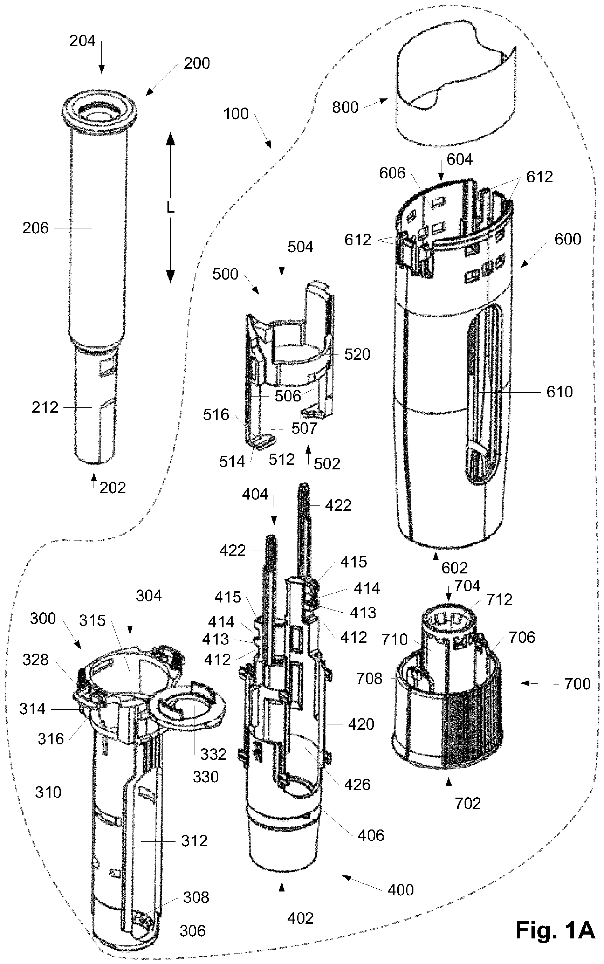

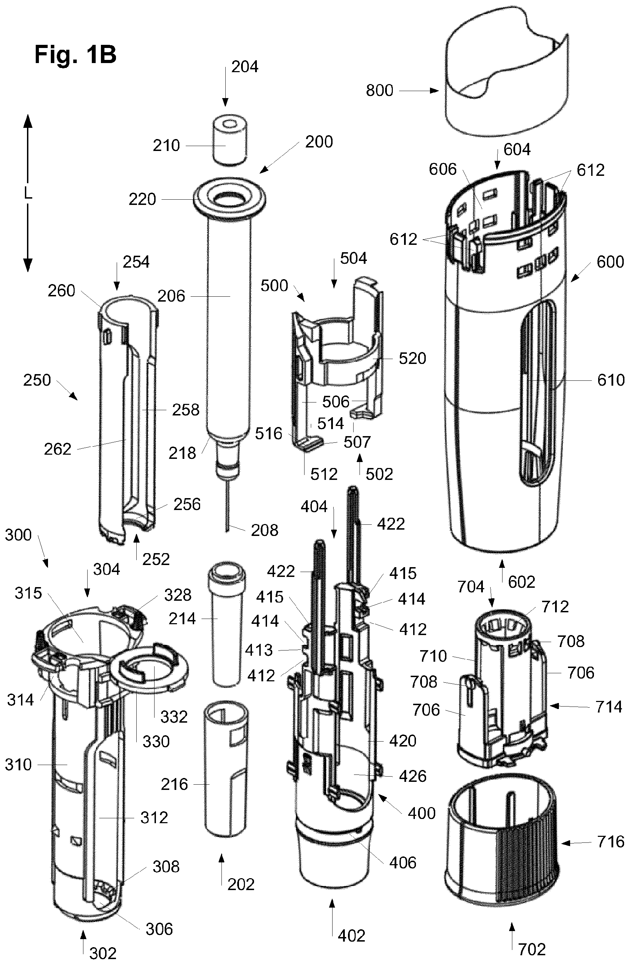

Figures 1a-b show exploded views of a cassette 100 extending along a longitudinal direction L, and a syringe 200 for being mounted in the cassette 100. The difference between figure 1a and figure 1b is in the size of the syringe 200, where the syringe 200 in figure 1a is larger than that shown in figure 1b. When a thin syringe 200 is to be included in the cassette 100, an additional syringe sleeve 250 is used for ensuring that the syringe 200 is sufficiently secured in the cassette 100 during use.

The syringe 200 extends from a proximal end 202 to a distal end 204. At the distal end 202 of the syringe 200 is a flange 220, which assists in securing the syringe 200 inside the cassette 100. The medicament is contained inside a syringe compartment 206, which is in fluid connection with a syringe outlet 208. Inside the syringe compartment 206 is a stopper 210, which pushes medicament out of the syringe outlet 208 when moving in the proximal direction. Normally, the stopper 210 is pushed in the proximal direction by means of a plunger rod included in an auto injector into which the cassette 100 with the syringe 200 is mounted.

In the shown examples, the syringe outlet 208 is a hollow needle attached to the syringe compartment 206. The needle 208 may be detachable to the syringe compartment by means or e.g. threaded connection or a Luer lock, or be an integral part of the syringe 200 as shown in figures 1a-b. The syringe compartment 206 has a shoulder 218 at its proximal connecting to the syringe outlet 208. Positioned around the syringe shoulder 218 is a needle shield 212, e.g. a rigid needle shield. The needle shield 212 will normally be constructed from two parts; an inner part 214 and an outer part 216 as shown in figure 1b. The needle shield 212 protects the needle prior to use.

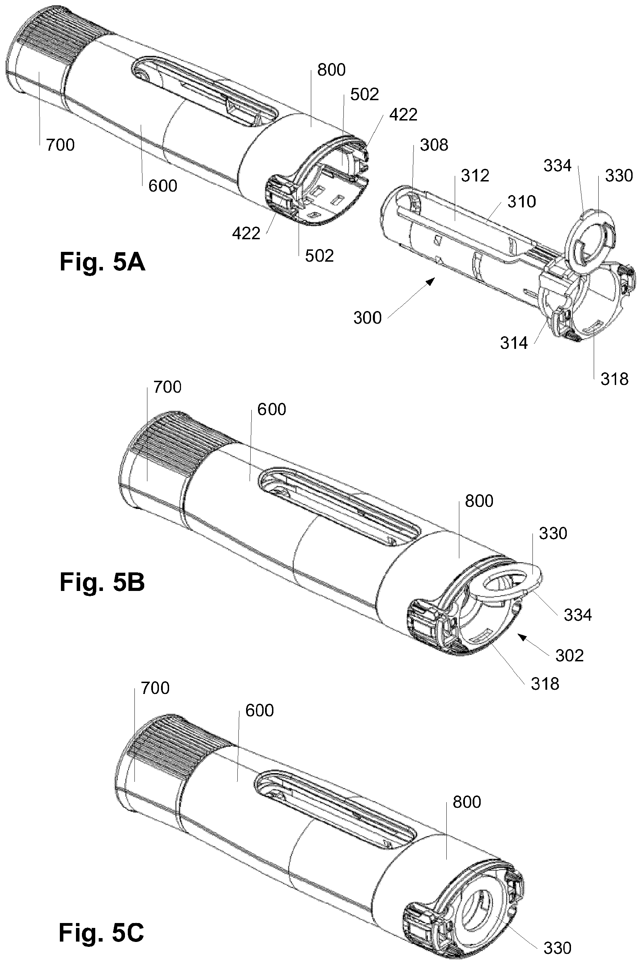

The syringe holder 300 extends from a proximal end 302 to a distal end 304 and is has an opening 306, 315 at both of these ends. The syringe 200 is mounted in the syringe holder 300 through the opening 315 at the distal end 304 of the syringe holder 300. When mounted in the syringe holder 300, the syringe compartment 206 is contained in a syringe holder tubular part 310 having a syringe holder tubular part inspection opening 312 through which the syringe 200 can be inspected. At the distal end 304 of the syringe holder 300 is a distal tubular ring part 314 having a larger outer diameter than that of the syringe holder tubular part 310. At the most distal end, there is a hinged syringe holder lid 330 with a lid opening 332 having a diameter, which allows passage of a plunger rod included in the auto injector there through. The syringe holder lid 330 is closed during assembly when a syringe 200 has been inserted into the cassette 100, as shown in figures 5a-c. A lid locking tab 334 on the syringe holder lid 330 locks inside a lid locking opening 318 on the distal tubular ring part 314, when the hinged syringe holder lid 330 is closed after assembly of a syringe 200 inside the syringe holder 300. Figure s 5a-c show the assembly of syringe holder 300 in the cassette 100 in figures 5a-b. Between the views in figure 5b and 5c, it is to be understood that a syringe 200 may be inserted in the syringe holder 300.

The cassette 100 may be fully assembled apart from the closing of the syringe holder lid 330 for easy delivery to a user. Alternatively, the syringe holder lid 330 may be opened again after transport for mounting of a syringe 200. The user may then insert the syringe 200 and close the syringe holder lid 330. Other means for securing the syringe 200 inside the syringe holder 300 may also be imagined. For example, the syringe holder lid 330 may be substituted with flexible arms, which flex inwardly to secure the syringe 200 after mounting of the same in the syringe holder 300.

The proximal opening 306 in the syringe holder 300 has a diameter, which allows the needle shield 212 to pass there through, but which prevents the syringe compartment 206 to pass there through. The proximal opening 207 has an inwardly extending support surface 308 for supporting the syringe shoulder 218.

The syringe sleeve 250 shown in figure 1b is used when the syringe compartment 206 has a diameter, which is similar to the diameter of the proximal opening 306 in the syringe holder 300. In order to ensure that the syringe 200 does not pass through the proximal opening 306, when e.g. the needles shield is removed, the syringe 200 is mounted in the syringe sleeve 250 prior to been positioned inside the syringe holder 300. The syringe sleeve 250 shown in figure 1b has a C-shape with a longitudinal mounting opening 258, which allows for sideways mounting of the syringe 200 in the syringe sleeve 250. At the proximal end 252 of the syringe sleeve 250 is an inward protruding support surface 256 against which the syringe should 218 rests. The needle shield 212 will be positioned outside the syringe sleeve 250 such that the inward protruding support surface 256 is placed between the needle shield 212 and the syringe shoulder 218. When mounted in the syringe sleeve 250, the syringe flange 220 will normally rest on a distal syringe sleeve support surface 260 at the distal end 254 of the syringe holder 250. The syringe 200 mounted in a syringe sleeve 250, may be positioned in a syringe holder 300 through the opening 315 in at the distal end 304 of the syringe holder 300 in the same manner as a larger syringe 200 is mounted into the syringe holder 300. When mounted in the syringe holder 300, the proximal end 252 of the syringe sleeve 250 is resting on the inwardly extending support surface 308 with the needle shield 212 extending in the proximal direction outside the syringe holder 300.

Apart for the syringe sleeve 250 and the diameter of the syringe 200, the remaining cassette parts shown in figure 1a and figure 1b are the same, and includes a cassette housing 600, a skin sensor 400, a cap 700, an index ring 500, and possibly a cassette housing cover 800. The cassette housing cover 800 may be formed as an integral part of the cassette housing 700.

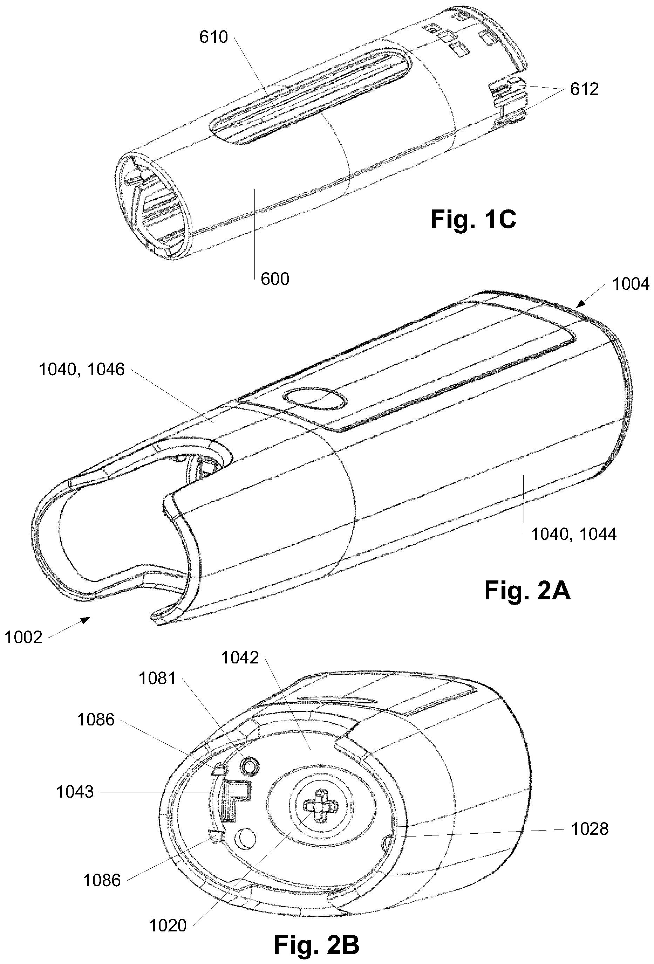

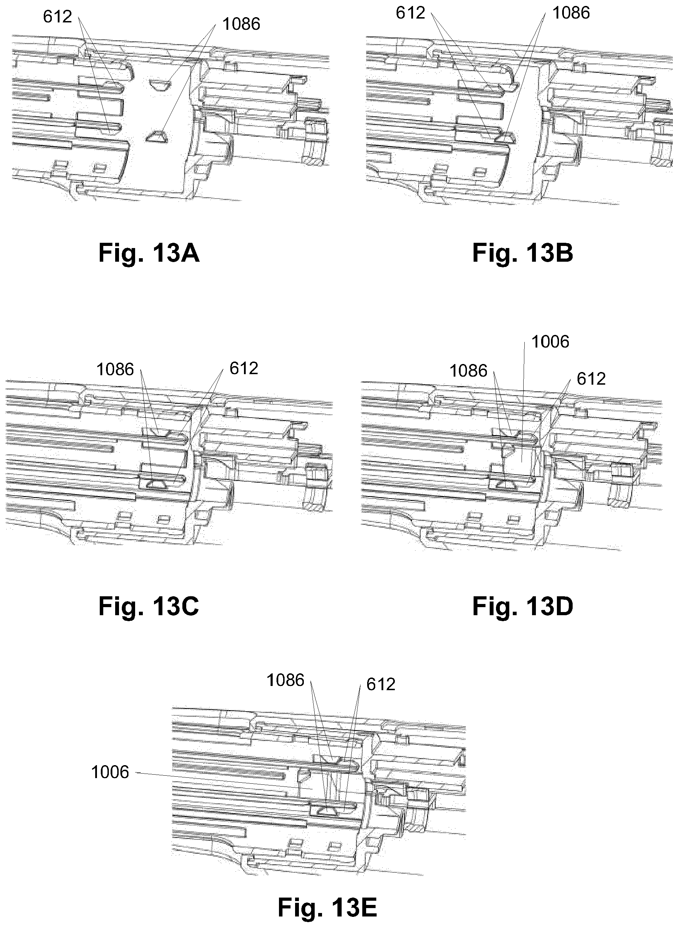

The cassette housing 600 extends from a proximal end 602 to a distal end 604 and has an opening 606 at the distal end 602 through which the index ring 500, the skin sensor 400, and the syringe holder 300 is mounted. When the cassette 100 is assembled, the cassette housing 600 at least partly if not fully encloses the syringe holder 300, the syringe 200 when positioned in the syringe holder 300, the skin sensor 400, and the index ring 500. The syringe holder 300 will normally be longitudinally and rotationally locked to the cassette housing 600. The cassette housing 600 has an inspection opening 610 on at least one surface side allowing the user to inspect the syringe compartment 206 containing the medicament when the syringe 200 is mounted inside the cassette 100. The cassette 100 may be symmetrically constructed such that it may be mounted in an auto injector in different orientations. At the distal end of the cassette housing, cassette securing tabs 612 are positioned. These are also visible in figure 1c showing the cassette housing 600 in a side-way view, where also the inspection opening 610 is clearly viewable. The locking mechanism is shown and described in figures 13a-e.

The cassette cap 700 is removably attached to the cassette housing 600 at the proximal end 602 of the cassette housing 600. The cassette cap 700 comprises an inner cassette cap part 714 with second distally extending part 710 in the form of a tubular cap part, which is configured for griping the needle shield 212 by means of e.g. a gripping portion 712 as shown in figures 1a-b. When removing the cassette cap 700, the needle shield 212 is also removed allowing for medicament delivery. Enclosing the inner cassette cap part 714 is an outer cassette cap part 716, which the user can hold when removing the cassette cap 700. The inner and the outer cassette cap parts 714, 716 may also be formed as one integral item. On the inner cassette cap part 716 is also found a cap locking part 706 with a cassette cap locking protrusion 708. The cap locking part 706 with the cassette cap locking protrusion 708 ensures that the cassette cap 700 is firmly secured to the cassette housing 600 before enabling the cassette 100 for cassette cap removal.

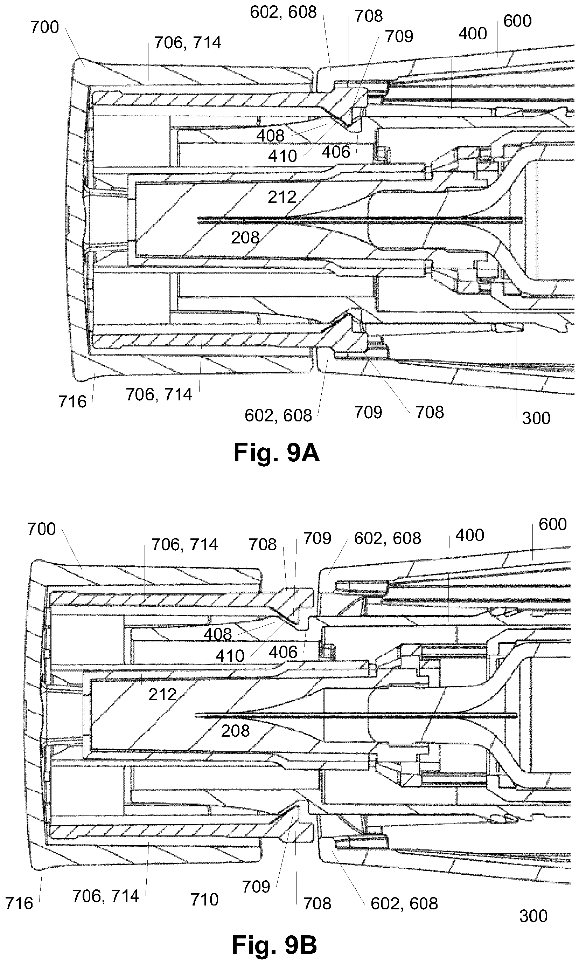

Positioned around the syringe holder 300 is a skin sensor 400, which extends from a proximal end 402 to a distal end 404. At the distal end 404 of the skin sensor 400 is a distal skin contact surface 405, which rest against the skin of the patient during medicament delivery. At the proximal end 404 is also a proximal recess 406 in which the cassette cap locking protrusion 708 of the cassette cap 700 is secured prior to assembling the cassette 100 in the auto injector 1000 and initiation release of the cassette cap 700 (see figures 9a-d). The proximal recess 406 is adjacent to a protruding tab 408 connected by a proximal protruding ramp 410 as more clearly seen in figures 9a-d.

The skin sensor 400 has a tubular skin sensor part 420 from where cassette skin sensor pins 422 extends in the distal direction. The tubular skin sensor part 420 has an inspection opening 426 for inspection of the syringe 200. The skin sensor 400 is normally symmetrically with a cassette skin sensor pin 422 on both sides of the skin sensor 400 and inspection openings 426 on both sides as well.

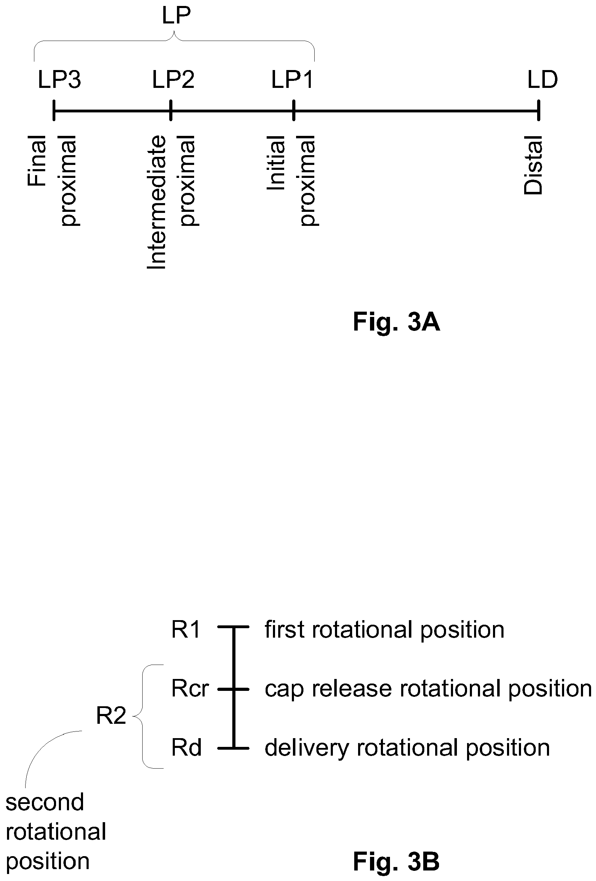

The skin sensor 400 is rotationally locked relative to the syringe holder 300. The skin sensor 400 is movable relative to the syringe holder 300 in the longitudinal direction L. The skin sensor 400 may move relative to the syringe holder 300 between a plurality of longitudinal positions including at least a proximal longitudinal position LP and a distal longitudinal position LD. As seen in figure 3a, three proximal positions LP are present apart from the distal position LD. The three proximal positions LP include an initial locked position LP1, an intermediate position LP2, and a final locked position LP3. The proximal positions LP are all positions, where the skin sensor 400 covers the syringe outlet 206 when a syringe 200 is mounted in the cassette 100, whereas the syringe outlet 206 is exposed when the skin sensor 400 is in the distal longitudinal position LD. The proximal positions LP1, LP2, LP3 may be positions in which the skin sensor 400 is locked relative to the syringe holder 300 in at least one longitudinal direction. Thus, in a locked position, proximal and/or distal movement of the skin sensor 400 relative to the syringe holder 300 is prevented. Whether the position is a locked position or not is determined by the rotational position of the index ring 500, as the skin sensor 400 and the index ring 500 are coupled such the rotation of the index ring 500 locks and releases the skin sensor 400 for longitudinal movement in either the proximal and/or the distal direction.

The skin sensor 400 also has a first locking recess 412 and a second locking recess 414 for interacting and coupling with the index ring 500. Between the two locking recesses 412, 412 is a first locking tab 413 and distally of the second locking recess 414 is a second locking tab 415. The second locking tab 415 is longer than the first locking tab 413. The distally directed surface of the second locking tab 415 is referred to as the first distal surface 416. The skin sensor 400 also comprises a proximally directed surface referred to as the first proximal surface 418. The first proximal surface 418 is proximally of the first locking recess 412.

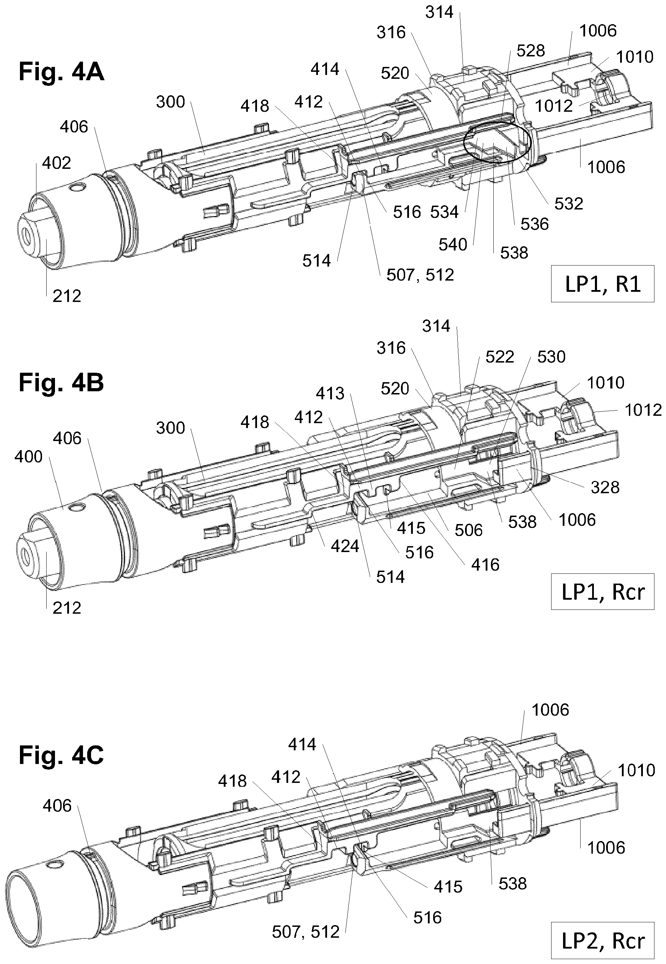

The index ring 500 extends from a proximal end 502 to a distal end 504. The index ring 500 is positioned around the syringe holder 300 and is longitudinally locked to the syringe holder 300. The index ring 500 is rotationally movable relative to the skin sensor 400 / syringe holder 300 between a plurality of rotational positions including at least a first rotational position R1 in which the skin sensor 400 is prevented from moving distally, and a second rotational position R2 in which the skin sensor 400 is moveable between the distal longitudinal position LD and the proximal longitudinal position LP. The rotational positions are illustrated in figure 3b, and as seen in figure 3b, the two rotational positions; the cap-release rotational position and the delivery rotational position both fall under the definition of a second rotational position R2 in which the skin sensor 400 is moveable between the distal longitudinal position LD and the proximal longitudinal position LP (see also the positions in figures 4b-e)

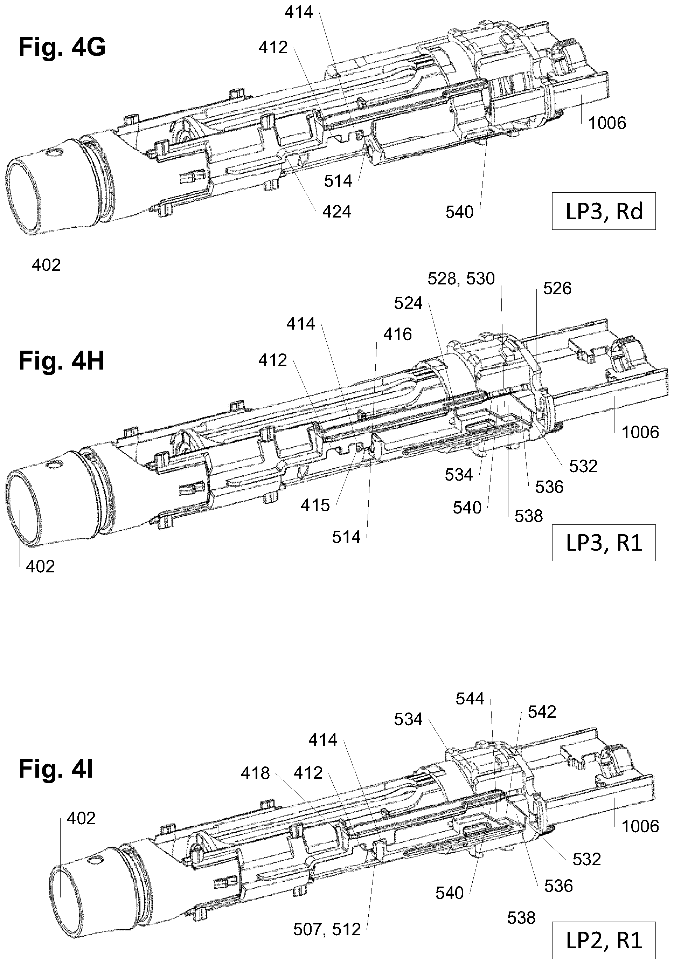

The index ring 500 has two first longitudinally extending part 506 positioned opposite each other on the index ring 500. A tubular index ring part 520 on the index ring 500 is extending around the syringe holder 300. The index ring 500 comprises a first radially extending part 522, which may be part of the first radially extending part 522 as shown most clearly in figures 4a-i. The first radially extending part 522 has a first surface area 528 extending between a first proximal surface 524 and a first distal surface 526 (see e.g. figure 4h). The first surface area 528 comprises a track 530 extending from a first corner 532 of the surface area 528 to a second corner 534 of the surface area 528 as shown in figure 4a. The surface area 528 on the first radially extending part 522 need not cover the entire surface of the first radially extending part 522, but can instead be a smaller area, which is defined by the by the two corners 532, 534 between which the track 530 extends.

The track 530 extends from the first corner 532 in an angled direction towards the second corner 534, wherein the angled directions is between 20 and 70 degrees, such as between 30 and 60 degrees, such as between 35 and 55 degrees, such as between 40 and 50 degrees, such as approximately 45 degrees relative to the longitudinal direction of the cassette. In the track 530m shown most clearly in figures 4a-i, the track 530 comprises a first track section 536, a second track section 538 and a third track section 540. The first track section 536 extends from the first corner 532 in a first angled direction towards a first middle point 542, the second track section 538 extends from the first middle point 542 to a second middle point 544 along the longitudinal direction of the cassette in a second angle direction, and the third track section 540 extends from the second middle point 544 in a third angled direction towards the second corner 534, as shown most clearly in figure 4i.

The first and/or third angled directions are normally sloping directions, with an angle between 20 and 70 degrees, such as between 30 and 60 degrees, such as between 35 and 55 degrees, such as between 40 and 50 degrees, such as approximately 45 degrees relative to the longitudinal direction of the cassette. The second track section 538 is normally a plateau track section with a second angled directions between -20 and 20 degrees, such as between -10 and 10 degrees, such as approximately 0 degrees relative to the longitudinal direction of the cassette.

On the first longitudinally extending part 506 of the index ring 500 is a second radially extending part 512. The second radially extending part 512 forms a locking tab 507, which is configured for fitting into the two locking recesses 412, 414 on the skin sensor 400. The locking tab 507 has a second proximal surface 514 and a second distal surface 516 of the second radially extending part 512.

The position of the locking tab 507 and thereby the rotational position of the index ring 500 relative to the skin sensor 400 is controlled by one or more release members 1006 in the auto injector 1000 when the cassette 100 is secured in the auto injector 1000. More specifically, the index ring 500 is configured for rotating from the first rotational position R1 to a second rotational position R2 when the cassette 100 is inserted in the auto injector 1000 and a track-guide protrusion 1008 comprised in the auto injector travels proximally inside the track 530. When the track-guide protrusion 1008 travels distally again, the index ring 500 rotates back form the second rotational position R2 to the first rotational position R1. Thus, by retrieving the release member 1006 / track-guide protrusion 1008 into the auto injector again, the index ring is rotated back to its initial position. As seen in figure 3b and figures 4be, the two rotational positions; the cap-release rotational position and the delivery rotational position both fall under the definition of a second rotational position R2 in which the skin sensor 400 is moveable between the distal longitudinal position LD and the proximal longitudinal position LP. Thus, the second rotational position R2 need not be a position from where the index ring 500 cannot rotate further. The second rotational position may also be an intermediate position in which the skin sensor 400 is unlocked, but in which the skin sensor 400 is still prevented from moving to the most proximal position and/or the most distal position possible.

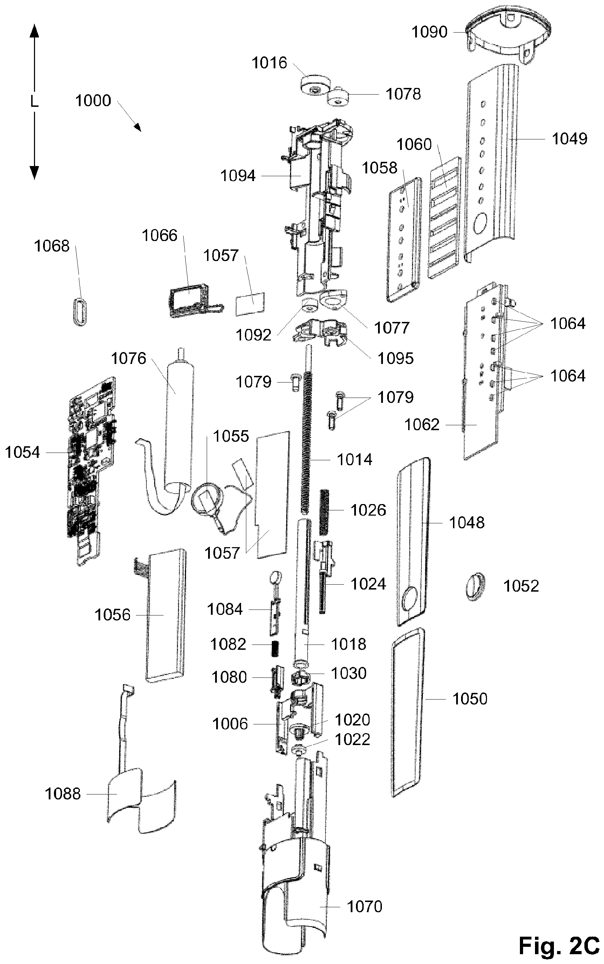

Figures 2a-b show the auto injector in an assembled view, and figure 2c shows an exploded view of the auto injector 1000 configured for receiving a cassette 100. The auto injector 1000 is extending from a proximal end 1002 to a distal end 1004 along a longitudinal axis L, and comprises an injector housing 1040 and a multiple of internal injector parts positioned inside the injector housing 1040.

The cassette 100 is removable received in the auto injector 1000 along the longitudinal direction L. Normally, the cassette 1000 will be received at the proximal end 1002 of the auto injector 1000, thereby making it a front-loaded auto injector. In figure 2b, a view inside the injector 1000 showing the cassette abutting surface 1042 where against which the cassette 100 is resting when assembled in the injector 1000 is shown. This view in figure 1b also shows the openings in the cassette abutting surface 1042 through which internal injector parts extend during medicament delivery and the preparatory step leading to the medicament delivery. The openings include a cassette detection pin opening 1081 through which a cassette detection pin 1080 extends, a skin sensor pin opening 1028 through which an injector skin sensor pin 1024 extends, and a release member opening 1043 through which a release member 1006 extends. Cassette locking tabs 1086 on the inside of the injector housing 1040 for securing the cassette 100 inside the auto injector 1000 can also be seen in figure 1b.

As seen most clearly in figure 1c, the internal injector parts includes a plunger rod 1018 configured for proximal movement for delivery of medicament. The plunger rod 1018 has an internal threaded surface, which is threadedly engaged with an outer threaded surface of a threaded rod 1014 positioned inside the plunger rod 1018. When the threaded rod 1014 inside the plunger rod 1018 rotates, the plunger rod 1018 is moved along the longitudinal axis L in the proximal or distal direction depending on which way the threaded rod 1014 rotates.

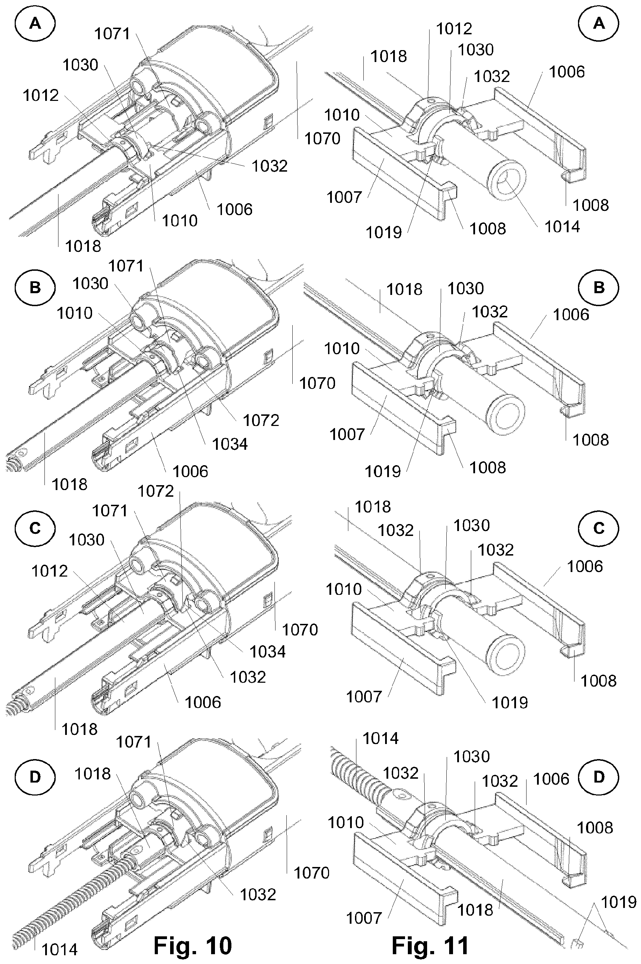

The auto injector 1000 comprises a release member 1006, which has two release member pins 1007 with each a track-guide protrusion 1008 (see also figure 11a-d). The release member 1006 travels inside the index ring track 530 thereby rotating the index ring 500, when the auto injector is activated after a cassette 100 is mounted in the auto injector 1000. The release member pins 1007 are connected by a release member bridge 1010 with a plunger rod opening 1012 allowing the plunger rod 1018 to pass through for medicament delivery.

The release member 1006 and the plunger rod 1018 are connected to a clutch 1030, which is configured for moving between a first clutch position where the plunger rod 1018 and the one or more release members 1006 are longitudinally coupled for proximal movement together; and a second clutch position where the plunger rod 1018 is decoupled from the one or more release members 1006 allowing the plunger rod 1018 to move longitudinally while the one or more release members 1006 are longitudinally stationary. This interaction is shown and discussed in more details in figures 10-12.

The auto injector 1000 further comprises an injector skin sensor pin 1024 and a skin sensor spring system 1026, which pushes the injector skin sensor pin 1024 proximally. When the cassette 100 is mounted in the auto injector 1000, the injector skin sensor pin 1024 will push the skin sensor 400 proximally when the skin sensor 400 is free to move proximally, and the user is not pressing the skin sensor in the distal direction simultaneously. Normally, the injector skin sensor pin 1024 will be abutting one of the cassette skin sensor pins 422 in the skin sensor 400.

Inside the auto injector 1000 is further a cassette detection pin 1080 and a cassette detection spring 1082, which detects when a cassette 100 has been mounted in the auto injector 1000 by making contact with the other cassette skin sensor pin 422 in the skin sensor 400.

As shown in figure 2a, the injector housing 1040 has a distal housing section 1044 and a cassette covering section 1046, where the distal housing section 1044 covers the internal injector parts and the proximal cassette covering section 1046 at least partly covers the cassette 100 when mounted in the auto injector 1000. The distal end 104 of the cassette 100 abuts a cassette abutting surface 1042 inside the auto injector 1000 when the cassette 100 is mounted in the auto injector 1000.

The internal injector parts also includes a drive module adapted for moving the plunger rod 1018 proximately. The drive module may be constructed in a number of manners of which the solution shown in figure 2c is only one example. The drive module shown in figure 2c includes a motor 1076 and motor gear wheels 1078 for rotating the threaded rod 1014 inside the plunger rod 1018, a battery 1056 e.g. chargeable from a USB charger accessible via the USB gasket 1068, and a number chassis parts 1070, 1077, 1090, 1094, 1095 and screws 1079 for supporting the drive module parts.

The auto injector 1000 further may include a processor controlling the drive module, the processor including a print circuit board (PCB) 1054. Visual indicating signals to the user may be included in the form of light emitting diodes (LEDs) 1064 possibly positioned on a PCB placeholder 1062 or similar. The LEDs are displayed via an LED window frame 1050, a light box placeholder 1058, and a light guide placeholder 1060.

The auto injector may be activated by pressing an activation button 1052 positioned on a user interface housing part 1048. Normally, a protective foil 1049 is included to protect the electronic parts inside the injector 1000. For the other internal injector parts, reference is made to the reference list.

Figures 4a-i show the syringe holder 300, the skin sensor 400, the index ring 500, and the injector release member 1006 in the different skin sensor longitudinal positions and index ring rotational positions as the injector release member 1006 moves longitudinally in the index ring track 530.

Figure 4a shows the initial locked position in which the cassette 100 is in prior to use. The locking tab 507 on the index ring 500 is secured in the first locking recess 412 on the skin sensor 400, which prevents longitudinal movement of the skin sensor 400 relative to the syringe holder 300. The skin sensor 400 is in the initial locked position LP1 and the index ring 500 is in the first rotational position R1. The first rotational position may also be seen as a locking rotational position, since this rotational position always locks the skin sensor 400 longitudinal in one of the proximal longitudinal positions.

Figure 4b shows the cap-release position in which the cassette cap 700 can be removed from the cassette 100 (see description of figures 9a-d for further details). Between figure 4a and 4b, the auto injector track-guide protrusion 1008 on the injector release member 1006 has moved into the index ring track 530 through the injector release member opening 322 and into and along the first track section 536 and is in the second plateau track section 538 in figure 4b. This has rotated the index ring 500 into the cap-release rotational position Rcr in which the locking tab 507 is released from the first locking recess 412 in the distal direction allowing the skin sensor to move proximally until the locking tab 507 abuts the second locking tab 415, which is longer than the first locking tab 413 securing the locking tab 507 inside the first locking recess 412 in figure 4a. Figure 4b shows the position of the skin sensor 400 after release, but before the skin sensor 400 is moved from the initial locked position LP1 to the ready-to-remove-cap position LP2.

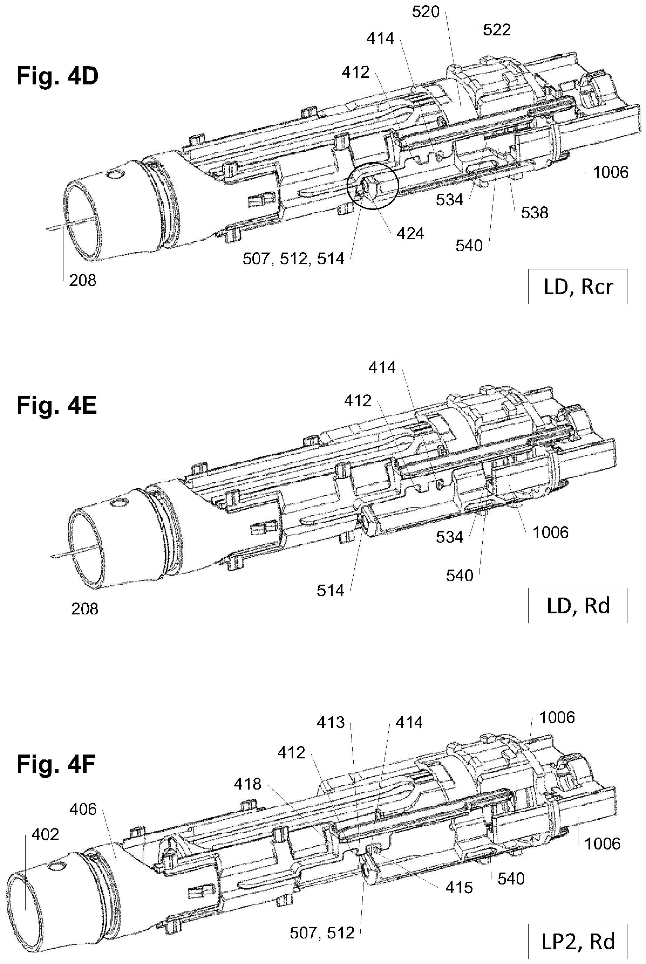

In figure 4c, the skin sensor 400 has moved proximately to the intermediate position LP2, where the cassette cap 700 can be removed. The index ring 500 is still in the cap-release rotational position Rcr, and the locking tab 507 abuts the second locking tab 415, which ensures that the skin sensor 400 is prevented from moving further in the proximal direction during cassette cap removal. The skin sensor 400 can however be moved in the distal direction by a user. This is shown in figure 4d, where the skin sensor is pushed into the distal longitudinal position LD where the needle 208 is no longer covered by the skin sensor 400 but instead inserted into an injector site. A second proximal surface 514 on the second radially extending part 512 / locking tab 507 abuts a second distal surface 424 on the skin sensor 400 therefore preventing further distal movement of the skin sensor 400.

When the skin sensor 400 is pushed into the distal longitudinal position LD as shown in figure 4d, the auto injector track-guide protrusion 1008 is ready to moved further proximally in the index ring track 530. When moving along the third track section 540, the index ring 500 is rotated to the delivery rotational position Rd as shown in figure 4e. When the auto injector track-guide protrusion 1008 reaches this position inside the index ring track 530, the injector release member 1006 is decoupled from the plunger rod 1018 by the clutch 1030 in the auto injector 1000. The plunger rod 1018 is thereby enabled for further proximal movement for medicament delivery while the injector release member 1006 remains in the position as shown in figure 4a.

After medicament delivery, the user removed the skin sensor from the skin and the injector skin sensor pin 1024 and skin sensor spring system 1026 ensures that the skin sensor is pushed first into the intermediate position LP2 as shown in figure 4f and finally into the final position LP3 as shown in figure 4g. When the skin sensor 400 is the final position LP3 as shown in figure 4g, the plunger rod 1018 moves distally and couples with the injector release member 1006 again when reaching the same coupling point as where it was decoupled during medicament delivery. The plunger rod 1018 and the injector release member 1006 moves together distally, which forces the index ring 500 to rotate from the delivery rotational position Rd into the first rotational position R1 again. When the index ring 500 is in the first rotational position R1 once more, the skin sensor 400 is locked longitudinally, this time in the final position LP3 as shown in figure 4h. The locking tab 507 now abuts a first distal surface 416 on the second locking tab 415.

Figure 4i shows the locked position, which the auto injector forces the cassette into if a user having removed the cassette cap when the cassette was in a configuration as shown in figure 4c, but for some reason have been prevented from continuing the medicament delivery process by inserting the needle into the injections site by pushing the skin sensor 400 distally. If this happens, the auto injector may be programmed to after a pre-determined time period move the injector release member 1006 distally, whereby the index ring 500 rotates into the first rotational position R1 thereby locking the skin sensor in the intermediate position LP2, where the locking tab 507 is secured in the second locking recess 414 on the skin sensor 400. The cassette 100 will be seen as being a used cassette if the user tries to mount it again the auto injector 1000, since the injection needle 208 may have been contaminated after removal of the needle shield 212 when removing the cassette cap 700.

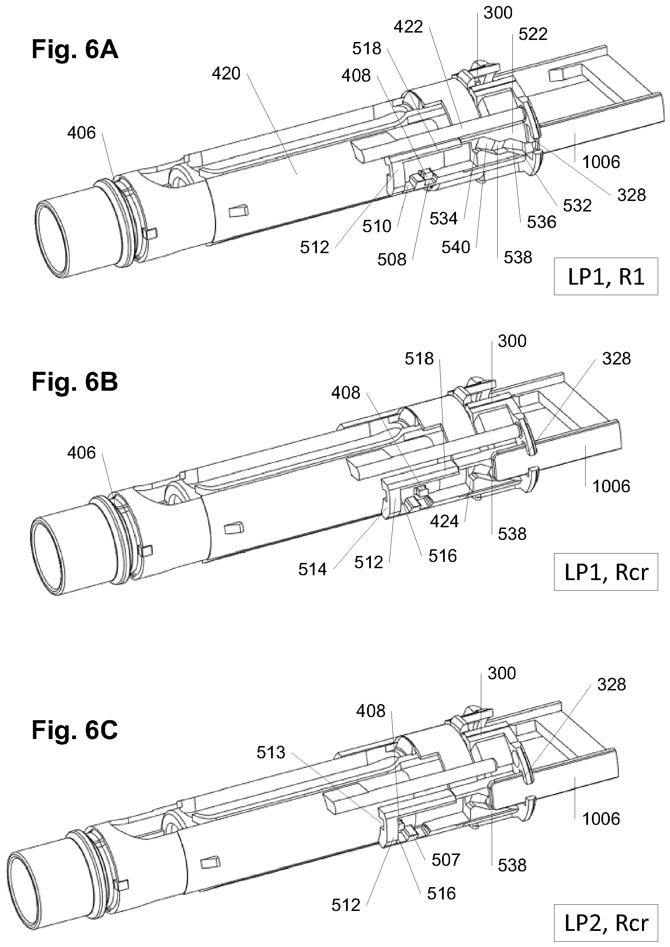

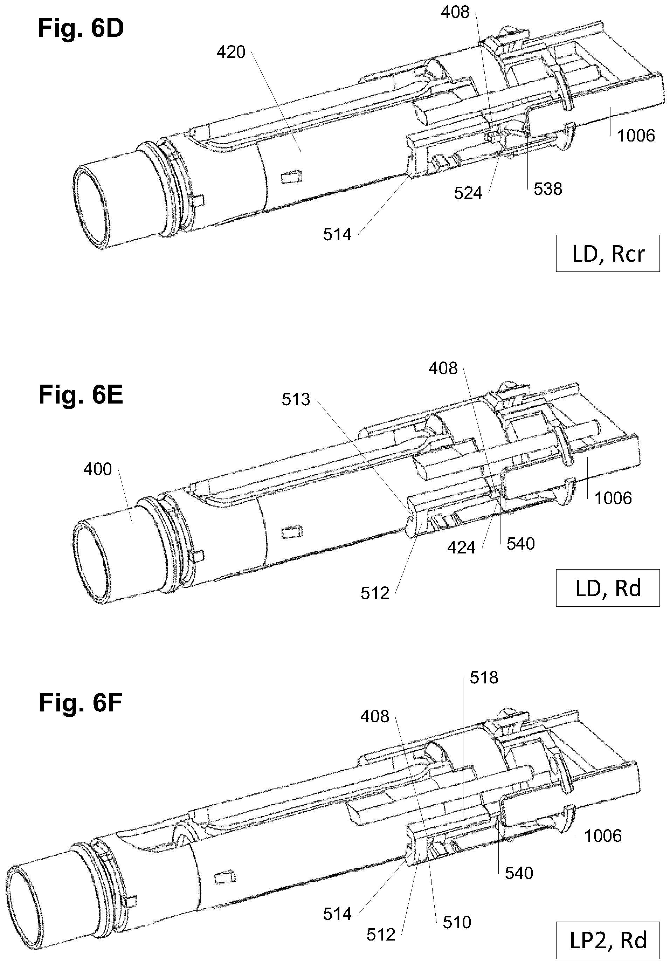

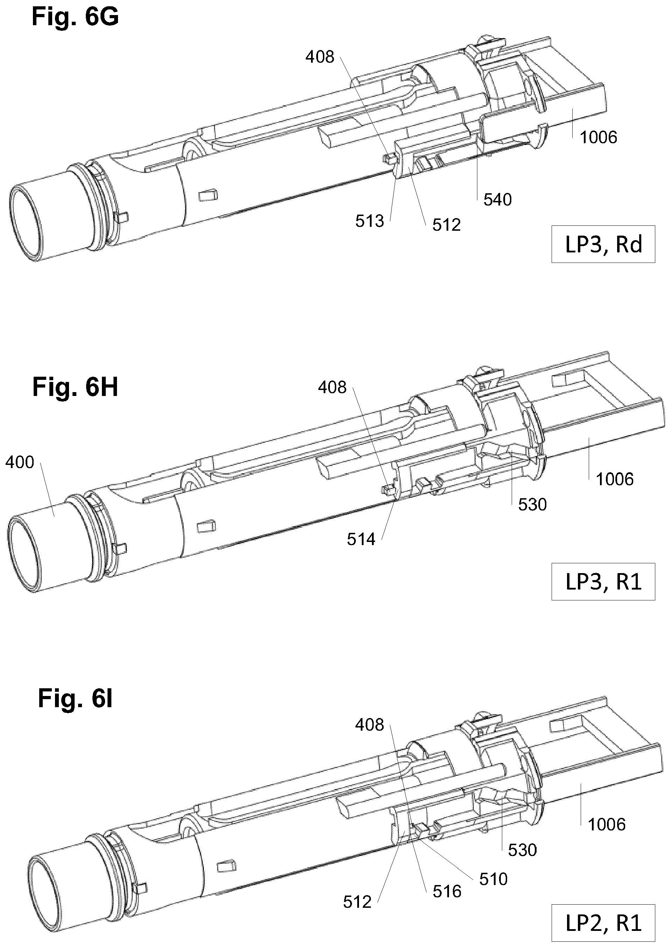

Figures 6i-a show a second example of the syringe holder 300, the skin sensor 400, the index ring 500, and the injector release member 1006 in the different skin sensor longitudinal positions and index ring rotational positions as the injector release member 1006 moves longitudinally in the index ring track 530. The positions which are shown are the same in the a-i figures for the examples shown in figure 4 and figure 6, i.e. figure 6a shows the initial locked position in which the cassette 100 is in prior to use; figure 6b shows the cap-release position; figure 6c shows the skin sensor in the intermediate position LP2, where the cassette cap 700 can be removed; figure 6d shows the position, where the skin sensor is pushed into the distal longitudinal position LD, but the index ring not completely rotated for delivery; figure 6e shows the delivery position; figure 6f shows an intermediate position after delivery; figure 6g shows the final position LP3 before locking; and figure 6h shows the locked final position. Figure 6i shows the locked position, which the auto injector forces the cassette into if a user having removed the cassette cap when the cassette was in a configuration as shown in figure 6c, but for some reason have been prevented from continuing the medicament delivery process by inserting the needle into the injections site by pushing the skin sensor 400 distally.

The differences between the 4a-i figures and the 6a-i figures are the design and form of the skin sensor 400 and the index ring 500. In figures 6a-i, the index ring 500 has a second longitudinally extending part 518 such that the longitudinal extending parts 516, 518 and the radially extending parts 512, 522 form an rectangle. Inside the rectangle on the first longitudinally extending part 506 is a first recess 508 and a second recess 510. These recesses are configured for containing a protruding tab 408 on the skin sensor 400. In the initial locked position LP1 as shown in figure 6a, the protruding tab 408 is contained inside the first recess 508. When the skin sensor 400 is in the intermediate position LP2, the protruding tab 408 is either in the second recess 510 if the index ring is rotated to the first rotational position as shown in figure 6i, or above the second recess 510, but supported in the proximal direction by the second distal surface 516 of the second radially extending part 512 thereby preventing further proximal movement during cap removal as shown in figure 6c. When the delivery of medicament occurs, the index ring 500 is rotated such that the protruding tab 408 is aligned with an opening 513 in the second radially extending part 512 such that when the skin sensor 400 is removed from the injection site, the protruding tab 408 is allowed to pass through the opening 513 in the second radially extending part 512 to the most proximal position, i.e. the final position LP3. This alignment is shown in figures 6e-g. When the index ring 500 is rotated back to the first rotational position R1, the protruding tab 408 is abutting the second proximal surface 514 of the second radially extending part 512 preventing the exposure of the needle 208 after medicament delivery as shown in figure 6h.

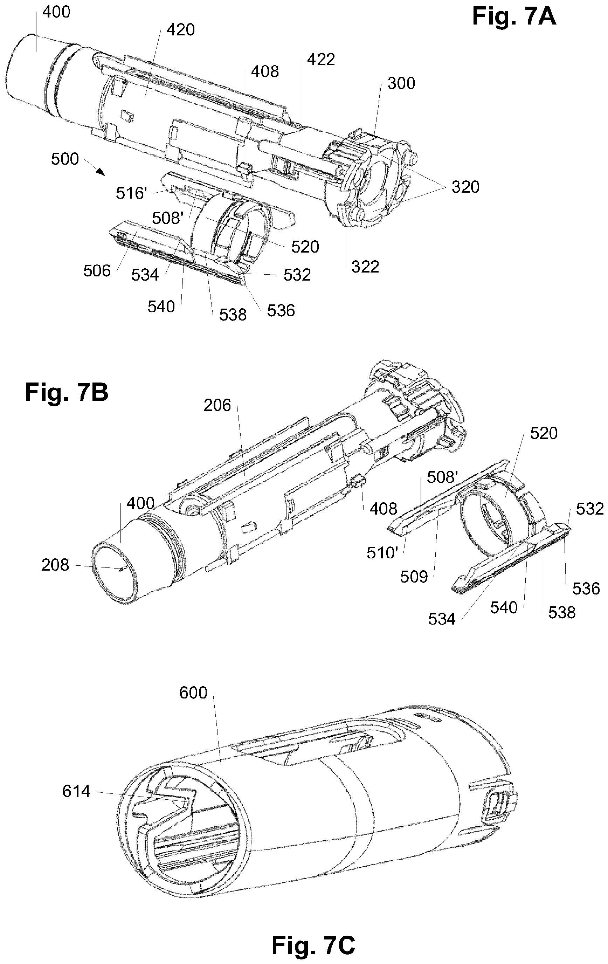

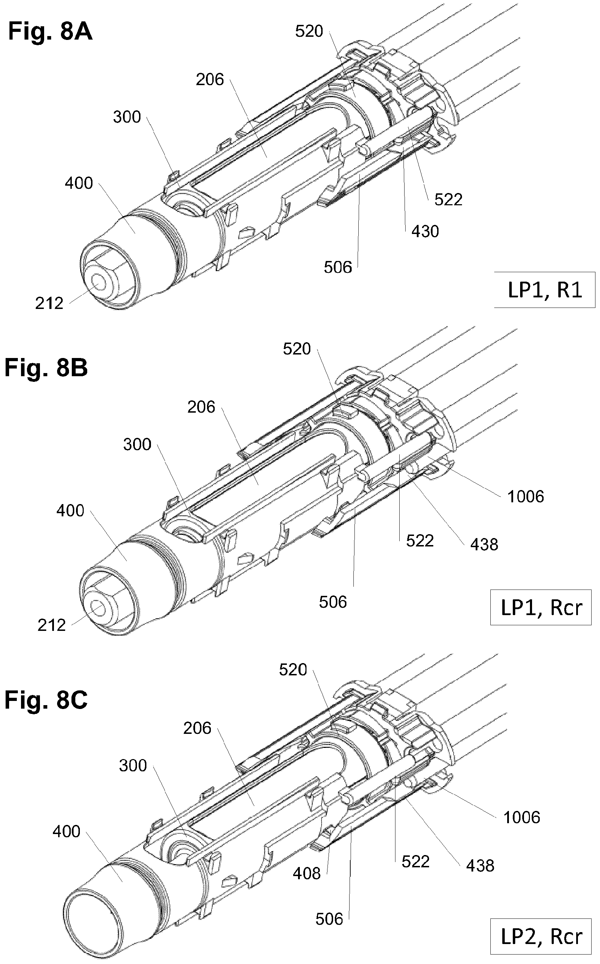

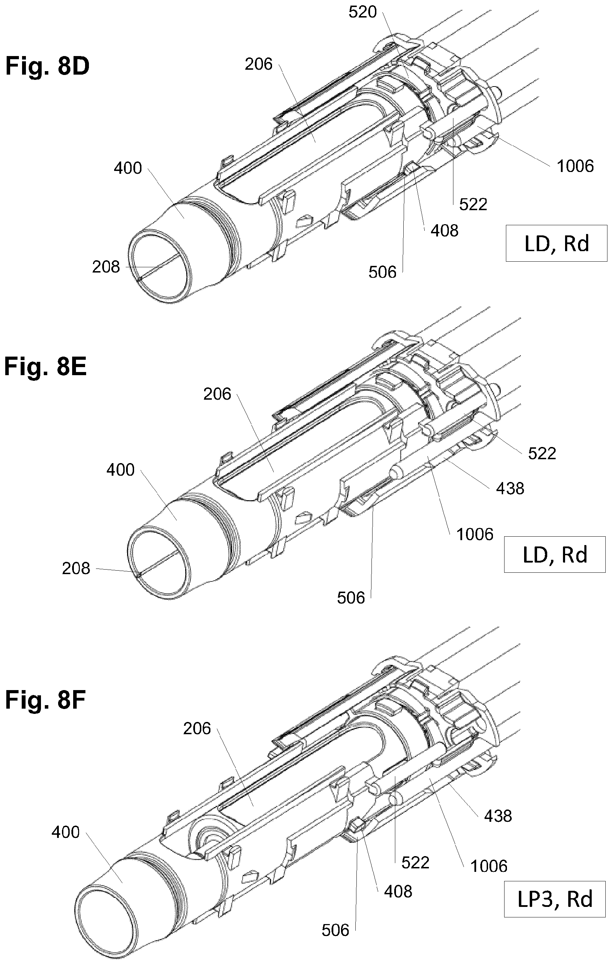

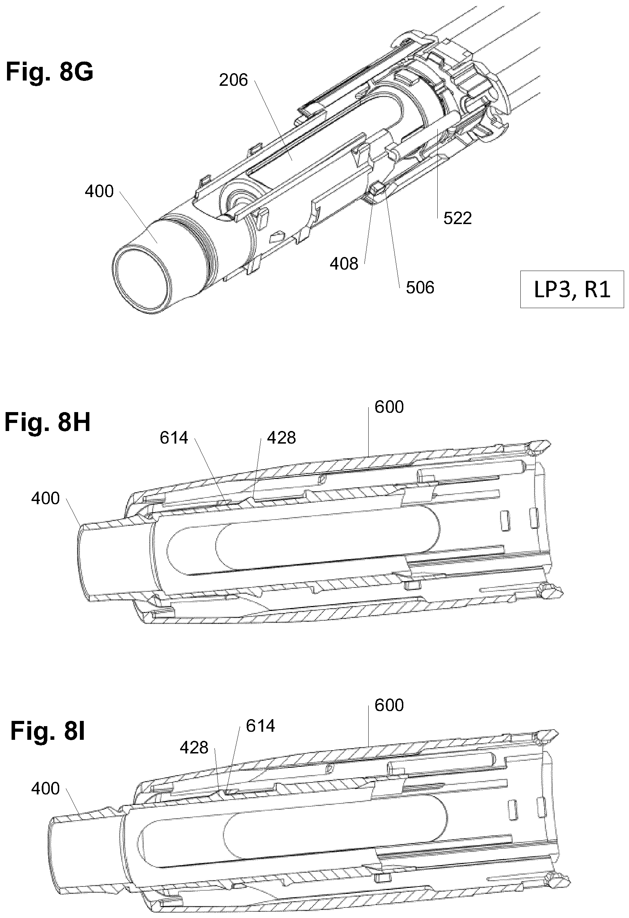

Figures 7 and 8 show a third example of the cassette, where figures 7a-b show the syringe holder 300, the skin sensor 400, the index ring 500, figure 7c shows the cassette housing 600, figures 8a-g show the syringe holder 300, the skin sensor 400, the index ring 500, and the injector release member 1006 in the different skin sensor longitudinal positions and index ring rotational positions as the injector release member 1006 moves longitudinally in the index ring track 530, and figures 8h-i show a cut-through of the cassette housing 600 and the skin sensor 400 showing the skin sensor 400 in non-locked position (figure 8h) and the final position LP3 (figure 8i).

The differences between figures 4a-i and 6a-i and figures 7a-c and 8a-i, are the design and form of the skin sensor 400 and the index ring 500, the shape of the release member 1006 in the injector 1000, and the locking of the skin sensor 400 in the final locked position LP3. In the third example, the track 530 is positioned on the first longitudinally extending part 506, which is attached to the tubular index part 520 on its outside. The track 530 is an open track. On an inner surface of the longitudinally extending part 506 are a first recess 508', a second recess 510' and an index ring ramp 509 extending distally from the first recess 508'. The skin sensor 400 has a protruding tab 408, which is confined in the first recess 508' prior to us as shown in figure 8a. After the index ring 500 is rotated to the cap-release rotational position Rcr position as shown in figure 8b, the skin sensor 400 moves to the cap-removal position shown in figure 8c. The cassette cap 700 and the needle shield 212 can be removed as shown in figure 8c. In the position shown in figure 8c, the protruding tab 408 rest against the second distal surface 516 of the second radially extending part 512. During distal movement of the skin sensor 400 by the user for insertion of the needle at the injection site, the index ring 500 is rotated to the delivery rotational position Rd as shown in figure 8d. Thus, in the third example shown in figures 7a-b and 8a-g, the release member 1006 is not the item rotating the index ring from the cap-release rotational position Rcr to the delivery rotational position Rd, but instead the protruding tab 408 coming in contact with the index ring ramp 509 thereby forcing the index ring 500 to rotate. During medicament delivery as shown in figure 8e, the release member 1006 moves further into the cassette 100. After end medicament delivery, the skin sensor 400 moves to the final position LP3 due to the spring force from the skin sensor pin spring system 1024, 1026 in the injector 1000. This is shown in figure 8f. In the position shown in figure 8g, the skin sensor 400 is in final locked position. In the final locked position LP3, the skin-sensor 400 is pushed proximally by a spring system as described in the previous examples of the cassette and auto injector and locked into a locking position in the cassette housing 600. The locking is obtained by a cassette housing locking protrusion 428 on the skin sensor 400 fitting into a skin sensor locking opening 614 in the cassette housing 600 as shown in figure 8i. In the position shown in figure 8h, the skin sensor 400 is in non-locked position. The skin sensor locking opening 614 in the cassette housing 600 is visible most clearly in figure 7c.

In figure 7a, an alternative securing of the syringe 200 inside the syringe holder 300 is also shown, where two flexible syringe securing tabs 320 moves from an open to a closed position securing the syringe 200 inside the syringe holder 300.

Figures 9a-d show the removal of the cassette cap 600 from the cassette 100. The cassette cap 700 has locking cap parts 706 extending distally from a proximal end 702 of the cassette cap 700. Prior to use, a cassette cap locking protrusion 708 on each of the one or more locking cap parts 706 is secured inside the cassette housing 600 by a cap holding part 608, which is extending radially inwardly at the proximal end 602 of the cassette housing 600. At the proximal end 402 of the skin sensor 400, the skin sensor 400 has a ramp 410 and a recess 406 positioned distally of the ramp 410. The ramp 410 normally forms a protruding tab 408. An inwardly extending part 709 of the cassette cap locking protrusion on each of the locking parts 706 are secured between the recess 406 of the skin sensor 400 and a cap holding part 608, and is further abutting the proximal protruding ramp part 410 preventing proximal movement of the cassette cap 600 prior to use as shown in figure 9a. This locks the cassette cap 700 to the cassette 100 and prevents that the cassette cap 700 is removed from the cassette 100 before the cassette 100 is assembled inside the auto injector 1000.

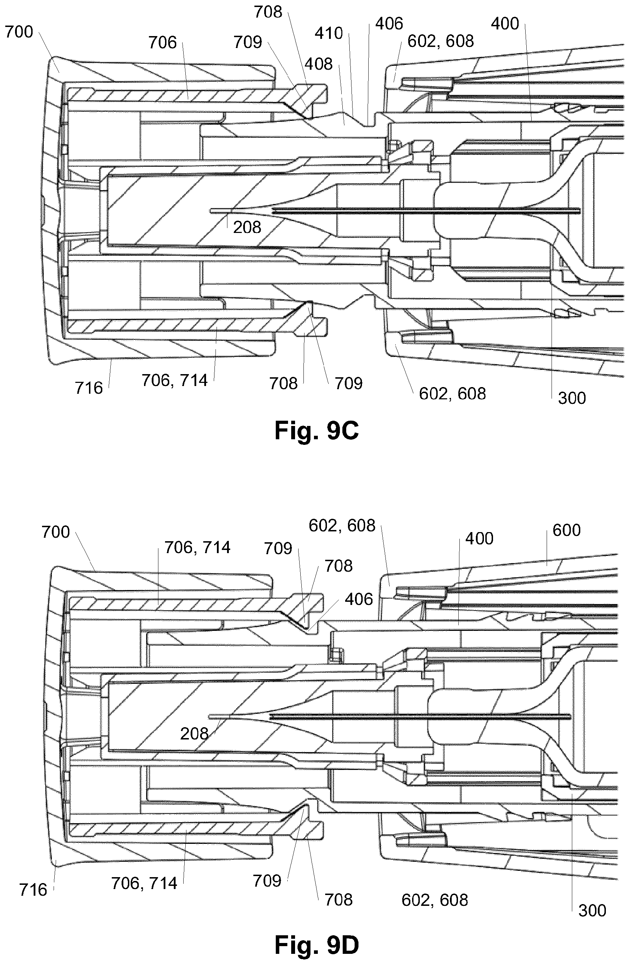

The cap locking parts 706 and/or the cassette cap locking protrusion 708 on each of the one or more cap locking part 706 are flexible. Upon proximal movement of the skin sensor 400 from the initial locked position LP1 to the intermediate position LP2, the cap locking parts 706 and/or the cassette cap locking protrusion 708 on the locking part 706 first flex slightly radially inwards in the recess 406 of the skin sensor 400 thereby enabling the cassette cap locking protrusion locking protrusions 708 to move proximally pass the cap holding part 608. When the skin sensor 400 is moved from the initial locked position LP1 to the intermediate position LP2, the distal locking ends 708 thereby move outside the cassette housing 600 as shown in figure 9b. Since the locking parts 706 and/or the cassette cap locking protrusion 708 on the locking part 706 are configured to flex, they now flex radially outwardly and slide up the ramp 410 as the user removes the cassette cap 700 from the cassette 100. This is shown in figure 9c. The cassette cap 700 with the needle shield 212 inside may be positioned on the skin sensor 400 after use to provide an additional security preventing access to the used needle. This is shown in figure 9d. The cassette cap 700 is not non-removably connected to the cassette 100 again, but it will stay in position unless pulled off again by a user.

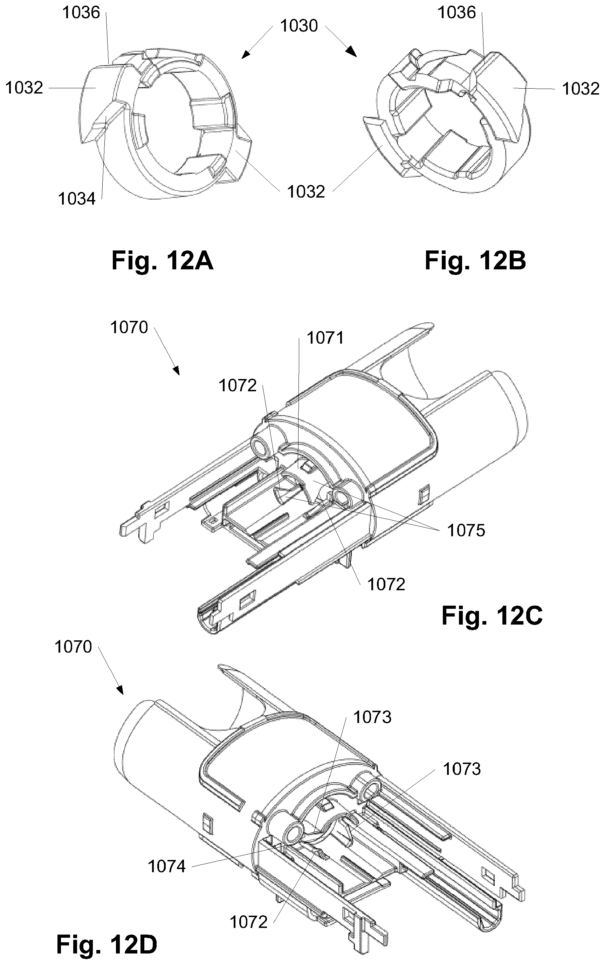

Figures 10a-d and 11a-d show the clutch coupling and decoupling in the injector 1000, figures 12a-b show the clutch 1030 in detail, and figures 12c-d show the proximal chassis part 1070 in details.

The clutch 1030 is positioned around the plunger rod 1018 and next to the release member 1006 such that in the first clutch position shown in figures 10a-b and 11a-b, the plunger rod 1018 and the release members 1006 are longitudinally coupled and move proximally together. The clutch 1030 is a tubular clutch having two clutch tabs 1032 each having a first rotation surface 1034 and a second rotation surface 1036. The proximal chassis part 1070 has a clutch coupling part 1071 with first coupling surfaces 1072 and second coupling surfaces 1073 on each of the small distally extending parts 1075.

When the second rotation surface 1036 on the clutch 1030 comes in contact with the second coupling surfaces 1073 on the clutch coupling part 1071, the clutch is rotated from the first clutch position and into the second clutch position as shown in figures 10c-d and 11c-d. In the second clutch position, the plunger rod 1018 is decoupled from the release members 1006 allowing the plunger rod 1018 to move longitudinally while the release members 1006 are longitudinally stationary. When the second rotation surface 1036 on the clutch is not in contact with the second coupling surfaces 1073 on the clutch coupling part 1071, the clutch is in the first clutch position. The clutch 1030 moves proximally with the release members 1006 before the second rotation surface 1036 comes into contact with the second coupling surfaces 1073, and axial rotates when the second rotation surface 1036 comes into contact with the second coupling surfaces 1073, thereby moving from the first clutch position to the second clutch position.

The clutch coupling part 1071 also comprises a clutch stopping surface 1074 abutting the clutch 1030 when the clutch 1030 is in the second clutch position thereby preventing the clutch 1030 and the release members 1006 positioned distally of the clutch 1030 from moving proximally with the plunger rod 1018.

The plunger rod 1018 comprises a stopping clutch tab 1019, and a distal surface of the stopping clutch tab 1019 is abutting a proximal surface of the release members 1006 when the clutch 1030 is in the first clutch position as shown in figures 10a-b and 11a-b. The stopping clutch tab 1019 rotates the clutch 1030 from the second clutch position to the first clutch position upon distal movement of the plunger rod 1018 after medicament delivery bringing the distal surface of the stopping clutch tab 1019 in contact with the proximal surface of the members 1006 again.

The clutch 1030 may be configured for moving from the first clutch position to the second clutch position when the plunger rod 1018 moves distally after medicament delivery. The clutch 1030 is in the first clutch position prior to use and normally moves to the second clutch position when the index ring 500 is also rotated to the delivery rotational position Rd by proximal movement of the one or more release members 1006.

When assembling the cassette 100 and the auto injector 1000, the cassette 100 is first loaded into the auto injector 1000. Upon loading of the cassette 100 in the auto injector 1000, cassette securing tabs 612 at the distal end 604 of the cassette housing 600 snap behind corresponding cassette securing openings 1028 in the injector housing 1040. By forward movement of the release member 1006, the cassette securing tabs 612 are prevented from deflecting and exiting the cassette securing openings 1028 in the injector housing 1040 thereby locking the cassette 100 to the auto injector 1000. As the cassette 100 is now firmly locked inside the auto injector 1000. When the cassette release member 1006 moves distally again, the cassette 100 is unlocked. The locking process is shown in figures 13a-e, where figures 13a-c show the cassette being inserted into the injector 1000 and engaging into a snap fit connection. Figures 13d-e show the movement of the release member 1006 into the cassette 100 thereby locking the cassette 100 to the injector 1000. Thus, the injector comprises one or more cassette interacting parts 1086 configured for engaging in a snap-fit connection with corresponding one or more cassette securing tabs 612 on the cassette 100 when the cassette is inserted into the injector, and wherein proximal movement of the release member 1006 prevents a disengagement of the snap-fit connection thereby securing the cassette inside the auto injector. The locking mechanism shown in figures 13a-e could alternatively be substituted with the similar locking turned 90 degrees.

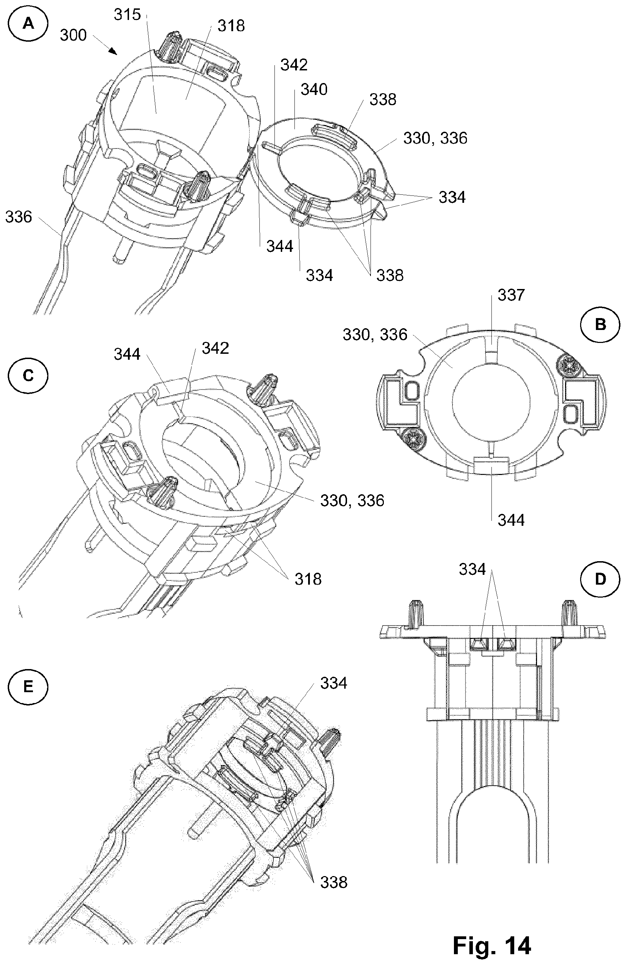

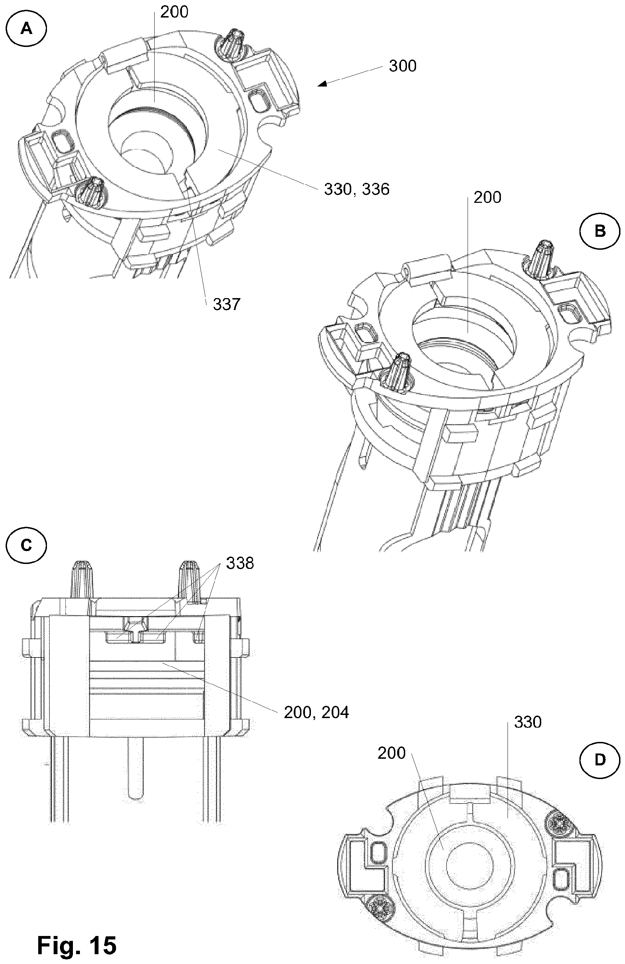

Figures 14a-e show an example of a syringe holder 300 suitable for use with any of the above described examples of the cassette 100. Figures 15a-d show the syringe holder 300 of figures 14a-e with a syringe 200 positioned inside the syringe holder 300. The syringe holder 300 is extending in a longitudinal direction L from a proximal end 302 to a distal end 304 as shown in figures 1a-b. The syringe holder 300 comprises the syringe holder tubular part 310 for receiving a syringe 200 and a syringe holder lid 330, which has a slightly different shape compared to that shown in the previous examples.

The holder lid 330 has a substantially C-shaped lid part 336 comprising an inner surface 340. The C-shaped lid part 336 may also be referred to as an open circle or a circle with a cut-through opening 337 in the circumference. The cut-through opening 337 may span an area of at least 10%, such as at least 20%, such as at least 30% of a full circular circumference. The substantially C-shaped lid part 336 is hingedly connected to the distal end of the syringe holder tubular part 310. By hingedly connected is meant that the substantially C-shaped lid part 336 can be in at least two positions as shown in figures 14a compared to figures 14c-e and figures 15a-d.

In figure 14a, the syringe holder lid 330 is in an open position allowing for a syringe 200 to be positioned in the syringe holder 300 through the opening 315 in the distal end of the syringe holder tubular part 310.

In figures 14c-e and figures 15a-d, the syringe holder lid 330 is in a closed position, where the syringe holder lid 330 prevents the syringe 200 from exiting the syringe holder 300 through the opening in the distal end of the syringe holder tubular part 310. This is also seen in figures 15a-d.

Extending radially outward from the substantially C-shaped lid part 336 are a plurality of lid locking tabs 334. These locking tabs 334 are positioned in corresponding syringe holder lid locking openings 318 in the syringe holder tubular part 310 when the substantially C-shaped lid part 336 is in the closed position as shown in figure 14b-e. Normally, each of the plurality of lid locking tabs 334 fits into a separate one of the syringe holder lid locking openings 318.

The inner surface 340 of the substantially C-shaped lid part 336 seen most clearly in figure 14a is oriented towards the syringe 200 when the syringe 200 is positioned in the syringe holder 300. Extending from the inner surface 340 is a plurality of support lid parts 338. In the example shown in figures 14-15, there are four support lid parts 338, but both more or less can be imagined. Compared to the entire inner surface 340 surface area, the support lid parts 338 cover an area of less than 50%, such as less than 40%, such as less than 30%, such as less than 20%, such as less than 10%. Having a broken surface structure on the surface, which the syringe may come in contact with ensures that if the user pushes on the syringe 200 unevenly from a proximal direction, not all of the support lid parts 338 will come in contact with the syringe. This ensures the pushing forces are not transferred around in the entire substantially C-shaped lid part 336. If a user should succeed in pushing so hard on the syringe 200 that one of the locking tabs 334 are pushed out of the syringe holder lid locking opening 318, there is an increased change that the other locking tabs 334 will remain in position thereby providing an increased security preventing the syringe 200 from being pushed out of the distal end of the syringe holder.

When the syringe 200 is correctly positioned inside the syringe holder tubular part 310 as shown in figures 15a-d, the syringe 200 is not in direct contact with the syringe holder lid 330. The syringe 200 only comes in contact with the syringe holder lid 330 if it is pushed distally during use. The syringe holder lid 330 therefore has a safety function and not a syringe fixation or syringe holding function. In figure 15c, the empty space between the distal end 204 of the syringe 200, i.e. the syringe flange, and the support lid parts 338 is seen most clearly.

The substantially C-shaped lid part 336 also has a throughgoing channel 342 in the dividing the substantially C-shaped lid part into two lid sections. The channel 342 does not divide the C-shaped lid part 336 into two completely separate parts, but instead the two sections are connected by a bridge portion 344 also forming the hinged connecting to the distal end of the syringe holder tubular part 310. This is seen most clearly in figure 14a-c. The first lid section and the second lid section are at least partly displaceable in relation to each other along the longitudinal axis. By this is meant that one section may be twisted or pushed distally or proximally without moving the other section to the same extend. The flexibility in the substantially C-shaped lid part 336 further ensures that if the user pushes on the syringe 200 unevenly from a proximal direction, only part of the substantially C-shaped lid part 336 is affected since the forces are not easily transferred around in the substantially C-shaped lid part 336.

The invention is further described in the following items
1. A cassette for use in an auto injector for administering a medicament, the cassette extending in a longitudinal direction (L) from a proximal end (102) to a distal end (104), the cassette (100) comprising:
   - a syringe holder (300) for receiving a syringe (200) containing the medicament;
   - a skin sensor (400) longitudinally movable relative to the syringe holder (300) between a plurality of longitudinal positions including at least a proximal longitudinal position (LP); and a distal longitudinal position (LD);
   - an index ring (500) rotatable relative to the skin sensor (400) between a plurality of rotational positions including at least a first rotational position (R1) in which the skin sensor (400) is prevented from moving distally; and a second rotational position (R2) in which the skin sensor (400) is moveable between the distal longitudinal position and the proximal longitudinal position (LP);
   wherein the rotational position of the index ring (500) is configured to be controlled by one or more release members (1006) in the auto injector when the cassette (100) is secured in the auto injector (1000).
2. The cassette according to any preceding item, wherein when the skin sensor (400) is in the proximal longitudinal position (LP), and the index ring is in the first rotational position, the skin sensor (400) is prevented from moving distally from the proximal longitudinal position (LP) by the index ring.
3. The cassette according to any preceding item, wherein prior to use, the index ring is in the first rotational position (R1) and the skin sensor (400) is locked relative to the syringe holder (300) in an initial locked position (LP1).
4. The cassette according to any preceding item, wherein when the index ring is in the second rotational position (R2), the skin sensor (400) is unlocked and able to move relative to the syringe holder (300) in a proximal direction and/or a distal direction.
5. The cassette according to any preceding item, wherein the index ring comprises a tubular index ring part (520) extending around a tubular skin sensor part (420).
6. The cassette according to any preceding item, wherein the index ring comprises a tubular index ring part (520) extending around the syringe holder (300).
7. The cassette according to any preceding item, wherein the skin sensor (400) comprises a protruding tab (408) on an outer surface of the skin sensor (400), and the index ring (500) comprises a first longitudinally extending part (506) with a first recess (508, 508') in which the protruding tab (408) is confined prior to use.
8. The cassette according to any preceding item, wherein the index ring (500) comprises a locking tab (507), and the skin sensor (400) comprises a first locking recess (412) in which locking tab (507) is confined prior to use.
9. The cassette according to any preceding item, wherein the index ring comprises a track (530) extending from a first corner (532) to a second corner (534), wherein the index ring (500) is configured for rotating from the first rotational position (R1) to the second rotational position (R2) when the cassette (100) is inserted in the auto injector (1000) and a release member (1006) comprised in the auto injector travels proximally inside the track (530).
10. The cassette according to item 9 wherein the index ring comprises a first radially extending part (522) having a first surface area (528) extending between a first proximal surface (524) and a first distal surface (526), wherein the track (530) is positioned on the surface area (528).
11. The cassette according to item 9, wherein the track (530) is positioned on the first longitudinally extending part (506).
12. The cassette according to any of the items 9-11, wherein the index ring is configured for rotating to the first rotational position when the release member (1006) comprised in the auto injector travels distally inside the track (530).
13. The cassette according to any of the items 10-12, wherein the first radially extending part (522) is part of the tubular index ring part (520).
14. The cassette according to any of the items 9-13, wherein the track (530) extends from the first corner (532) in an angled direction towards the second corner (534), wherein the angled directions is between 20 and 70 degrees, such as between 30 and 60 degrees, such as between 35 and 55 degrees, such as between 40 and 50 degrees, such as approximately 45 degrees relative to the longitudinal direction of the cassette.
15. The cassette according to item 14, wherein the track (530) comprises a first track section (536), a second track section (538) and a third track section (540), wherein:
   ∘ the first track section (536) extends from the first corner (532) in a first angled direction towards a first middle point (542);
   ∘ the second track section (538) extends from the first middle point (542) to a second middle point (544) along the longitudinal direction of the cassette in a second angle direction;
   ∘ the third track section (540) extends from the second middle point (544) in a third angled direction towards the second corner (534),
   wherein the first and/or third angled directions are between 20 and 70 degrees, such as between 30 and 60 degrees, such as between 35 and 55 degrees, such as between 40 and 50 degrees, such as approximately 45 degrees relative to the longitudinal direction of the cassette, and
   wherein the second angled directions is between -20 and 20 degrees, such as between -10 and 10 degrees, such as approximately 0 degrees relative to the longitudinal direction of the cassette.
16. The cassette according to any preceding item, wherein prior to use, the skin sensor (400) is locked in a initial locked position (LP1), and after use, the skin sensor is in a final locked position (LP3), wherein the initial locked position (LP1) is positioned between a final locked position (LP3) and the distal longitudinal position (LD).
17. The cassette according to any preceding item, wherein the skin sensor (400) comprises a protruding tab (408) on an outer surface of the skin sensor (400), and the index ring (500) comprises a first longitudinally extending part (506) with a first recess (508, 508') in which the protruding tab (408) is confined prior to use.
18. The cassette according to any preceding item, wherein the index ring (500) comprises a locking tab (507), and the skin sensor (400) comprises a first locking recess (412) in which locking tab (507) is confined prior to use.
19. The cassette according to any of the items 17-18, wherein the first longitudinally extending part (506) extends proximally from the first proximal surface (524) on the first radially extending part (522).
20. The cassette according to any of the items 17-19, wherein the protruding tab (408) abuts the first proximal surface (524) when the skin sensor (400) is in the distal longitudinal position (LD) thereby preventing distal movement of the skin sensor (500) relative to the syringe holder (300).
21. The cassette according to any of the items 18-19, wherein the skin sensor (400) comprises a second distal surface (424), wherein the second distal surface (424) abuts the second proximal surface (514) of the second radially extending part (512) when the skin sensor (500) is in the distal longitudinal position (LD) thereby preventing distal movement of the skin sensor (500) relative to the syringe holder (300).
22. The cassette according to any of the items 17-20, wherein the index ring (500) further comprises a second radially extending part (512) having a second proximal surface (514) and a second distal surface (516, 516').
23. The cassette according to item 22, wherein the protruding tab (408) is abutting the second proximal surface (514) when the skin sensor is in the final locked position (LP3) and the index ring (500) is in the first rotational position (R1) thereby preventing distal movement of the skin sensor relative to the syringe holder.
24. The cassette according to item 22, wherein the distal surface (416) is abutting the second proximal surface (514) when the skin sensor is in the final locked position (LP3) and the index ring (500) is in the first rotational position (R1) thereby preventing distal movement of the skin sensor relative to the syringe holder.
25. The cassette according to any of the items 22-24, wherein the second radially extending part (512) is connected to a proximal end of the first longitudinally extending part (506).
26. The cassette according to any of the items 22-25, wherein the index ring (500) comprises a second longitudinally extending part (518) extending from the first proximal surface (524) of the first radially extending part (522) and connected to the second radially extending part (512) at a proximal end of the second longitudinally extending part (518).
27. The cassette according to any of the items 3-26, wherein the index ring (500) is configured for being rotated to a cap-release rotational position (Rcr) positioned between the first rotational position (R1) and a delivery rotational position (Rd), wherein in the cap-release rotational position (Rcr) the skin sensor is movable between the distal longitudinal position (LD) and an intermediate position (LP2), wherein the intermediate position (LP2) is positioned between the final locked position (LP3) and the initial locked position (LP1).
28. The cassette according to item 27, wherein the index ring (500) is in the cap-release rotational position (Rcr) when the track-guide protrusion (1008) comprised in the auto injector is in the second track section (538).
29. The cassette according to any of the items 27-28, wherein when the index ring is in the cap-release rotational position (Rcr), the skin sensor (400) is prevented from moving from the intermediate position (LP2) to the final locked position (LP3).
30. The cassette according to any of the items 27-29, wherein when the index ring is in the cap-release rotational position (Rcr) and the skin sensor (400) is in the intermediate position (LP2), the protruding tab (408) abuts the second distal surface (516, 516') on the index ring.
31. The cassette according to any of the items 27-29, wherein when the index ring is in the cap-release rotational position (Rcr) and the skin sensor (400) is in the intermediate position (LP2), the locking tab (507) on the index ring (500) abuts a second locking tab (415) on the skin sensor (400).
32. The cassette according to any of the items 27-31, wherein the skin sensor (400) is configured for being moved to the distal longitudinal position (LD) by a user pushing the skin sensor (400) against an injection site when the index ring is:
   - in the cap-release rotational position (Rcr), or
   - between the cap-release rotational position (Rcr) and the delivery rotational position (Rd), or
   - in the delivery rotational position (Rd).
33. The cassette according to any preceding item, wherein the cassette further comprises a syringe (200) having:
   - a syringe compartment (202) containing the medicament;
   - an outlet (204) at a proximal end (202) of the syringe compartment;
   - a stopper (210) positioned inside the syringe compartment, wherein the stopper is movable from a distal end (204) of the syringe compartment towards the proximal end of the syringe compartment for emptying the medicament in the syringe compartment through the outlet;
   wherein when the skin sensor (400) is in the final locked position (LP3) and the index ring (500) is in the first rotational position (R1), the skin sensor (400) covers the syringe outlet (204), and
   wherein when the skin sensor (400) is in the distal longitudinal position (LD) and the index ring (500) is in the second rotational position (R2), the syringe outlet (204) is exposed.
34. The cassette according to item 33, wherein medicament can be delivered by an auto injector induced proximal movement of the stopper (210) inside the syringe compartment (206) when the skin sensor (400) is in the distal longitudinal position (LD) and the index ring is in the delivery rotational position (Rd).
35. The cassette according to any of the items 27-34, wherein the first longitudinally extending part (506) comprising a second recess (510, 510') in which the protruding tab (408) is confined when the skin sensor is in the intermediate position (LP2) and the index ring is in the first rotational position (R1).
36. The cassette according to any of the items 27-34, wherein the protruding tab (507) on the first longitudinally extending part (506) is confined in a second locking recess (414) on the skin sensor when the skin sensor is in the intermediate position (LP2) and the index ring is in the first rotational position (R1).
37. The cassette according to any preceding item, wherein the index ring (500) is longitudinally locked relative to the syringe holder (300).
38. The cassette according to any preceding item, wherein the skin sensor (400) and the syringe holder (300) are rotationally locked.
39. The cassette according to any preceding item, wherein the cassette further comprises a cassette housing (600) and a removable cassette cap (700) positioned at a proximal end (602) of the cassette housing (600), wherein the cassette housing (600) at least partly encloses the syringe holder (300), the syringe (200) when positioned in the syringe holder (300), the skin sensor (400), and the index ring (500) at least prior to use.
40. The cassette according to item 39, wherein the cassette cap (700) comprises one or more cap locking parts (706) extending distally from a proximal end (702) of the cassette cap (700), wherein prior to use, a cassette cap locking protrusion (708) on each of the one or more cap locking parts (706) is secured inside the cassette housing (600).
41. The cassette according to item 40, wherein:
   - the skin sensor (400) comprises a recess (406) and ramp (410) at the proximal end of the skin sensor (400), the recess (406) being positioned distally of the ramp (410); and
   - the cassette housing (600) comprising a cap holding part (608) extending radially inwardly at the proximal end (602) of the cassette housing (600);
   wherein prior to removal of the cassette cap (700), an inwardly extending part (709) of the cassette cap locking protrusion (708) on each of the one or more cap locking parts (706) is secured between the recess (406) of the skin sensor and the cap holding part (608), and is further abutting the proximal protruding part (410) preventing proximal movement of the cassette cap (600).
42. The cassette according to any of the items 40-41, wherein upon proximal movement of the skin sensor from the initial locked position (LP1) to the intermediate position (LP2), the cassette cap locking protrusions (708) are moved to a position outside the cassette housing (600), and wherein when the skin sensor is in the intermediate position (LP2), removal of the cassette cap (700) is possible.
43. The cassette according to any of the items 39-42, wherein the syringe (200) comprises a removable needle shield (212) covering the syringe outlet (208) prior to use, and wherein the cassette cap comprises a second distally extending part (710) extending around and grapping the needle shield (212), thereby removing the needle shield (212) when the cassette cap (700) is removed from the cassette housing (600).
44. The cassette according to any of the items 33-43, wherein the syringe outlet is a hollow needle.

### References

- 100: cassette
- 102: proximal end of the cassette
- 104: distal end of the cassette
- 200: syringe
- 202: proximal end of the syringe / proximal end of the syringe compartment
- 204: distal end of the syringe / distal end of the syringe compartment
- 206: syringe compartment
- 208: hollow needle / syringe outlet
- 210: stopper
- 212: needle shield / RNS
- 214: inner part of the needle shield
- 216: outer part of the needle shield
- 218: syringe flange
- 220: syringe shoulder
- 250: syringe sleeve
- 252: proximal end of the syringe sleeve
- 254: distal end of the syringe sleeve
- 256: syringe sleeve inward protruding support surface
- 258: C-shaped longitudinal mounting opening
- 260: distal syringe sleeve support surface
- 300: syringe holder
- 302: proximal end of the syringe holder
- 304: distal end of the syringe holder
- 306: proximal opening in the syringe holder
- 308: syringe support surface
- 310: syringe holder tubular part
- 312: syringe holder tubular part inspection opening
- 314: distal tubular ring part
- 315: opening in the distal end of the syringe holder
- 316: proximal index ring support surface
- 318: syringe holder lid locking opening
- 320: syringe securing flexible tab at the distal end of the syringe holder
- 322: injection release member opening
- 330: syringe holder lid
- 332: syringe holder lid opening
- 334: lid locking tab
- 336: C-shaped lid part
- 337: opening in the C-shaped lid part
- 338: support lid part
- 340: inner surface on the C-shaped lid part
- 342: C-shaped lid part channel
- 344: bridge portion
- 400: skin sensor
- 402: proximal end of the skin sensor
- 404: distal end of the skin sensor
- 405: distal skin contact surface of the skin sensor
- 406: proximal recess
- 408: protruding tab
- 410: proximal protruding ramp
- 412: first locking recess on the skin sensor
- 413: first locking tab
- 414: second locking recess on the skin sensor
- 415: second locking tab
- 416: first distal surface
- 418: first proximal surface
- 420: tubular skin sensor part
- 422: cassette skin sensor pin
- 424: second distal surface
- 426: inspection opening in the skin sensor
- 428: cassette housing locking protrusion
- 500: index ring
- 502: proximal end of the index ring
- 504: distal end of the index ring
- 506: first longitudinally extending part
- 507: locking tab
- 508, 508': first recess on the index ring
- 509: index ring ramp
- 510, 510': second recess on the index ring
- 512: second radially extending part
- 513: opening in the second radially extending part
- 514: second proximal surface of the second radially extending part
- 516, 516': second distal surface of the second radially extending part
- 518: second longitudinally extending part
- 520: tubular index ring part
- 522: first radially extending part
- 524: first proximal surface of the first radially extending part
- 526: first distal surface of the first radially extending part
- 528: first surface area of the first radially extending part
- 530: track in the first radially extending part
- 532: first corner of the first radially extending part
- 534: second corner of the first radially extending part
- 536: first section of the track
- 538: second section of the track
- 540: third section of the track
- 542: first middle point of the track
- 544: second middle point of the track
- 600: cassette housing
- 602: proximal end of the cassette housing
- 604: distal end of the cassette housing
- 606: opening at the distal end of the cassette housing
- 608: cap holding part
- 610: inspection opening
- 612: cassette securing tabs
- 614: skin sensor locking opening
- 700: cassette cap
- 702: proximal end of the cassette cap
- 704: distal end of the cassette cap
- 706: cap locking part
- 708: cassette cap track-guide protrusion / cassette cap locking protrusion
- 709: inwardly extending part of the cassette cap locking protrusion
- 710: second distally extending part / tubular cap part
- 712: gripping portion
- 714: inner cassette cap part
- 716: outer cassette cap part
- 800: cassette housing cover
- 1000: auto injector
- 1002: proximal end of the auto injector
- 1004: distal end of the auto injector
- 1006: release member
- 1007: release member pin
- 1008: track-guide protrusion
- 1010: release member bridge
- 1012: plunger rod opening
- 1014: threaded rod
- 1016: motor and threaded rod connector
- 1018: plunger rod
- 1019: stopping clutch tab
- 1020: first plunger rod plug part
- 1022: second plunger rod plug part engaging the stopper
- 1024: injector skin sensor pin
- 1026: skin sensor spring system
- 1028: skin sensor pin opening in the cassette abutting surface
- 1030: clutch
- 1032: clutch tab
- 1034: first rotation surface on the clutch tab
- 1036: second rotation surface on the clutch tab
- 1040: injector housing
- 1042: cassette abutting surface
- 1043: release member opening in the cassette abutting surface
- 1044: distal housing section
- 1046: cassette covering section
- 1048: first user interface housing part
- 1049: protective foil
- 1050: LED window frame
- 1052: activation button
- 1054: Print circuit board (PCB)
- 1055: RCT battery
- 1056: battery
- 1057: adhesive
- 1058: light box placeholder
- 1060: light guide placeholder
- 1062: PCB placeholder
- 1064: LED placeholder with an LED
- 1066: content management system (CMS)
- 1068: USB gasket
- 1070: proximal chassis part
- 1071: clutch coupling part
- 1072: first coupling surface on the clutch coupling part
- 1073: second coupling surface on the clutch coupling part
- 1074: clutch stopping surface on the clutch coupling part
- 1076: motor
- 1077: motor flange
- 1078: motor gear
- 1079: screw
- 1080: cassette detection pin
- 1081: cassette detection pin opening in the cassette abutting surface
- 1082: cassette detection spring
- 1084: cassette detect holder
- 1086: cassette locking tab in the injector housing
- 1088: NFC antenna for detection of the RFID chip on the cassette
- 1090: distal end part connecting to the housing
- 1092: bearing
- 1094: middle chassis part
- 1095: distal chassis part

- L: longitudinal direction
- LD: distal longitudinal position / delivery position
- LP: proximal longitudinal position
- LP1: initial locked position
- LP2: intermediate position / ready-to-remove-cap position
- LP3: final locked position
- R1: first rotational position / locking rotational position
- R2: second rotational position
- Rcr: cap-release rotational position
- Rd: delivery rotational position

## Claims

1. A cassette for use in an auto injector for administering a medicament, the cassette extending in a longitudinal direction (L) from a proximal end (102) to a distal end (104), the cassette (100) comprising:
• a syringe holder (300) for receiving a syringe (200) containing the medicament;
• a skin sensor (400) longitudinally movable relative to the syringe holder (300) between a plurality of longitudinal positions including at least a proximal longitudinal position (LP); and a distal longitudinal position (LD);
• an index ring (500) rotatable relative to the skin sensor (400) between a plurality of rotational positions including at least a first rotational position (R1) in which the skin sensor (400) is prevented from moving distally; and a second rotational position (R2) in which the skin sensor (400) is moveable between the distal longitudinal position and the proximal longitudinal position (LP);
wherein the rotational position of the index ring (500) is configured to be controlled by one or more release members (1006) in the auto injector when the cassette (100) is secured in the auto injector (1000).

2. The cassette according to any preceding claim, wherein when the skin sensor (400) is in the proximal longitudinal position (LP), and the index ring is in the first rotational position, the skin sensor (400) is prevented from moving distally from the proximal longitudinal position (LP) by the index ring.

3. The cassette according to any preceding claim, wherein prior to use, the index ring is in the first rotational position (R1) and the skin sensor (400) is locked relative to the syringe holder (300) in an initial locked position (LP1).

4. The cassette according to any preceding claim, wherein when the index ring is in the second rotational position (R2), the skin sensor (400) is unlocked and able to move relative to the syringe holder (300) in a proximal direction and/or a distal direction.

5. The cassette according to any preceding claim, wherein:
• the skin sensor (400) comprises a protruding tab (408) on an outer surface of the skin sensor (400), and the index ring (500) comprises a first longitudinally extending part (506) with a first recess (508, 508') in which the protruding tab (408) is confined prior to use; or
• the index ring (500) comprises a locking tab (507), and the skin sensor (400) comprises a first locking recess (412) in which locking tab (507) is confined prior to use.

6. The cassette according to any preceding claim, wherein the index ring comprises a track (530) extending from a first corner (532) to a second corner (534), wherein the index ring (500) is configured for rotating from the first rotational position (R1) to the second rotational position (R2) when the cassette (100) is inserted in the auto injector (1000) and a release member (1006) comprised in the auto injector travels proximally inside the track (530).

7. The cassette according to claim 6, wherein the index ring is configured for rotating to the first rotational position when the release member (1006) comprised in the auto injector travels distally inside the track (530).

8. The cassette according to any of the claims 6-7, wherein the track (530) extends from the first corner (532) in an angled direction towards the second corner (534), wherein the angled directions is between 20 and 70 degrees, such as between 30 and 60 degrees, such as between 35 and 55 degrees, such as between 40 and 50 degrees, such as approximately 45 degrees relative to the longitudinal direction of the cassette.

9. The cassette according to claim 8, wherein the track (530) comprises a first track section (536), a second track section (538) and a third track section (540), wherein:
∘ the first track section (536) extends from the first corner (532) in a first angled direction towards a first middle point (542);
∘ the second track section (538) extends from the first middle point (542) to a second middle point (544) along the longitudinal direction of the cassette in a second angle direction;
∘ the third track section (540) extends from the second middle point (544) in a third angled direction towards the second corner (534),
wherein the first and/or third angled directions are between 20 and 70 degrees, such as between 30 and 60 degrees, such as between 35 and 55 degrees, such as between 40 and 50 degrees, such as approximately 45 degrees relative to the longitudinal direction of the cassette, and
wherein the second angled directions is between -20 and 20 degrees, such as between -10 and 10 degrees, such as approximately 0 degrees relative to the longitudinal direction of the cassette.

10. The cassette according to any preceding claim, wherein prior to use, the skin sensor (400) is locked in a initial locked position (LP1), and after use, the skin sensor is in a final locked position (LP3), wherein the initial locked position (LP1) is positioned between a final locked position (LP3) and the distal longitudinal position (LD).

11. The cassette according to any preceding claim, wherein:
• the skin sensor (400) comprises a protruding tab (408) on an outer surface of the skin sensor (400), and the index ring (500) comprises a first longitudinally extending part (506) with a first recess (508, 508') in which the protruding tab (408) is confined prior to use, and/or
• the index ring (500) comprises a locking tab (507), and the skin sensor (400) comprises a first locking recess (412) in which locking tab (507) is confined prior to use.

12. The cassette according to any of the claims 3-11, wherein the index ring (500) is configured for being rotated to a cap-release rotational position (Rcr) positioned between the first rotational position (R1) and a delivery rotational position (Rd), wherein in the cap-release rotational position (Rcr) the skin sensor is movable between the distal longitudinal position (LD) and an intermediate position (LP2), wherein the intermediate position (LP2) is positioned between the final locked position (LP3) and the initial locked position (LP1).

13. The cassette according to claim 12, wherein when the index ring is in the cap-release rotational position (Rcr), the skin sensor (400) is prevented from moving from the intermediate position (LP2) to the final locked position (LP3).

14. The cassette according to any of the claims 12-13, wherein the skin sensor (400) is configured for being moved to the distal longitudinal position (LD) by a user pushing the skin sensor (400) against an injection site when the index ring is:
• in the cap-release rotational position (Rcr), or
• between the cap-release rotational position (Rcr) and the delivery rotational position (Rd), or
• in the delivery rotational position (Rd).

15. The cassette according to any preceding claim, wherein the index ring (500) is longitudinally locked relative to the syringe holder (300), and/or the skin sensor (400) and the syringe holder (300) are rotationally locked.
